# EUROPEAN PATENT APPLICATION

(11) **EP 4 177 900 A2**
(43) Date of publication of application: **10.05.2023**
(21) Application number: 22201130.6
(22) Date of filing: 12.10.2022
(51) Int. Cl.: G16B 30/00, G16B 35/20

(54) **LEVENSHTEIN DISTANCE-BASED IRES SCREENING METHOD AND POLYNUCLEOTIDE SCREENED BASED ON SAME**

(30) Priority: 12.10.2021 CN 202111185073; 29.11.2021 CN 202111435528
(71) Applicant: PURECODON (HONG KONG) BIOPHARMA LIMITED, North Point (HK)
(72) Inventor: HOU, Qiangbo, Suzhou, 215123 (CN); ZHU, Jiafeng, Suzhou, 215123 (CN); QIU, Zonghao, Suzhou, 215123 (CN); ZUO, Chijian, Suzhou, 215123 (CN); SUN, Zhenhua, Suzhou, 215123 (CN)
(74) Representative: Heubeck, Christian

(57) **Abstract**

The disclosure belongs to the technical field of bioinformatics and bioengineering, and specifically, relates to a Levenshtein distance-based IRES screening method, a polynucleotide screened based on this method, a circular nucleic acid molecule including the polynucleotide, a cyclization precursor nucleic acid molecule, a recombinant nucleic acid molecule, a recombinant expression vector, a recombinant host cell, and use. In the disclosure, averages of Levenshtein distances between all sample sequences and to-be-predicted sequences are compared, to efficiently and accurately determine whether there is an IRES in the to-be-predicted sequence, which has advantages of high efficiency and an accurate screening result. In addition, the IRES screened by the IRES prediction method provided by the disclosure has high activity, thereby providing abundant translation initiation elements for application of the circular nucleic acid molecule in preparing a protein, serving as vaccines, producing a therapeutic protein, or serving as a means of gene therapy, etc.

## Description

### TECHNICAL FIELD

The disclosure belongs to the technical field of bioinformatics and bioengineering, and specifically, the disclosure relates to use of a polynucleotide in initiating translation of a circular nucleic acid molecule, a polynucleotide having an activity of initiating translation of a circular nucleic acid molecule, a circular nucleic acid molecule including the polynucleotide, a cyclization precursor nucleic acid molecule, a recombinant nucleic acid molecule, a recombinant expression vector, a recombinant host cell, and use.

### BACKGROUND

A messenger ribonucleic acid (mRNA) is transcribed from DNA and provides genetic information required for the next protein translation. When mRNA for encoding an antigenic protein is injected into the human body, the antigenic protein can be synthesized in the body, thereby inducing intense cellular and humoral immune responses and showing a characteristic of an autoimmune adjuvant, which makes the mRNA an excellent vaccine means. In addition, the mRNA has many other advantages as a vaccine or for production of a therapeutic protein. For example, compared with a DNA vector, the mRNA is transiently expressed in cells, without a risk of integration into a genome or dependence on a cell cycle, and therefore, the mRNA is much safer; compared with a viral vector, the mRNA does not have a feature of immune resistance caused by the vector itself, and therefore, protein is easier to express; and compared with a recombinant protein, a virus, and the like, a cell-free system is used during a production process of the mRNA, which only involves an in vitro enzyme-catalyzed reaction, resulting in a simpler and more controllable production process with lower costs. Currently, the mRNA shows a wide range of application potentials in serving as the vaccine, producing the therapeutic protein, serving as a means of gene therapy, and the like.

Currently, mRNAs for clinical or preclinical use are mainly linear mRNAs, and a structure of the linear mRNA includes a 5' cap structure, a 3' polyadenosine tail (PolyA tail), a 5' untranslational region (5' UTR), a 3' untranslational region (3' UTR), an open reading frame (ORF), and the like. The 5' cap structure is an essential feature of eukaryotic mRNA and is obtained by adding N7-methylguanosine to a 5' end of the mRNA. Studies have shown that the 5' cap structure is bound to a translation initiation complex eif4E to promote mRNA translation, and can effectively prevent mRNA degradation and reduce immunogenicity of the mRNA. A main function of the 3' polyadenosine tail is to bind to polyA binding protein (PABP) that interacts with eiF4G and eiF4E to mediate formation of circular mRNA, promote the translation, and prevent the mRNA degradation. The 5' and 3' untranslational regions, such as 5' and 3' untranslational regions using beta-globin, can effectively prevent mRNA degradation and promote translation from the mRNA to the protein.

Circular RNAs (circRNAs) are a common type of RNAs in eukaryotes. Natural circRNAs are mainly produced through a molecular mechanism referred to as "back splicing" in cells. Currently, it has been found that eukaryotic circRNAs have a variety of molecular and cellular regulatory functions. For example, the circular RNA can be bound to microRNAs (miRNAs) to regulate expression of target genes; and the circular RNA can be directly bound to a target protein to regulate gene expression, and the like. Currently identified circular RNAs mainly function as non-coding RNAs. However, circular RNAs capable of encoding proteins also exist in nature, namely, circular mRNAs. The circular mRNAs tend to have a longer half-life due to their circular properties, and therefore, it is speculated that the circular mRNAs may be more stable. Methods of forming the circular RNA in vitro include a chemical method, a protease catalysis method, a ribozyme catalysis method, and the like.

An internal ribosome entry site (IRES) is a cis-acting RNA sequence capable of recruiting ribosomal subunits to a translation initiation site of the mRNA independently of the 5' cap structure, to mediate translation processes of viruses, some eukaryotes, and the like. The circular RNAs have a closed ring structure and lack typical translation initiation elements, but the circular RNAs can still implement a translation function by mediating the binding of ribosomes to the mRNAs by using the IRESs. Compared with linear mRNA, circular mRNA molecules have high stability and have important application prospects in protein expression and clinical treatment. A protein expression level of the circular mRNA molecules is affected by the translation initiation element. Therefore, finding more IRES elements that can initiate translation of the circular mRNA molecules is of great significance for improvement of the protein expression level of the circular mRNA molecules and expansion of application of the circular mRNA molecules to clinical and industrial production.

Currently, because confirmation, mechanism of action studies and structure studies of the IRESs in sequences mainly rely on experimental verification and it takes a lot of time and costs to screen out active IRES sequences from a large number of sequences with unknown functions, currently, a few IRESs are discovered and verified, which limits the application of the circular RNA molecules in protein expression, clinical treatment, and the like.

### SUMMARY

### Problems to be solved in the present invention

In view of the problems existing in the prior art, for example, the screening of sequences containing an IRES is time-consuming and costly, resulting in a small number of verified IRES sequences at present, which limits the application of circular mRNA molecules in protein expression, clinical treatment, etc. For this purpose, the disclosure provides a Levenshtein distance-based IRES screening method, which can efficiently and rapidly screen a to-be-predicted sequence containing the IRES, and the screening results are accurate, which is conducive to the discovery of new IRES sequences.

In some embodiments, the disclosure provides a polynucleotide including any one nucleotide sequence shown in (i), where the polynucleotide is capable of initiating a translation process of a circular nucleic acid molecule, has high IRES activity, and is capable of improving the protein expression level of the circular nucleic acid molecule, which provides abundant translation initiation elements for the further application of the circular nucleic acid molecule.

### Solutions for solving the problems

According to a first aspect, the disclosure provides a Levenshtein distance-based IRES screening method, including the following steps:
(1) selecting n sequences including an IRES as sample sequences, where n≥1 and n is a natural number;
(2) subjecting the sample sequences and to-be-predicted sequences to one-hot encoding respectively, where categorical variables are A, T, C, and G;
(3) traversing the sample sequences, and calculating a Levenshtein distance between each sample sequence and the to-be-predicted sequence;
(4) calculating an average of Levenshtein distances between all sample sequences and the to-be-predicted sequences; and
(5) determining, based on the average, whether the to-be-predicted sequences include the IRES.

In some embodiments, according to the Levenshtein distance-based IRES screening method in the disclosure, in the step (5), if the average is not less than a set prediction threshold, it is determined that the to-be-predicted sequence includes the IRES, otherwise it is determined that the to-be-predicted sequence includes no IRES.

In some embodiments, according to the Levenshtein distance-based IRES screening method in the disclosure, the prediction threshold is not less than 0.5.

In some embodiments, according to the Levenshtein distance-based IRES screening method in the disclosure, the prediction threshold is 0.75.

In some embodiments, according to the Levenshtein distance-based IRES screening method in the disclosure, the method further includes the following step of: traversing sample sequences if the to-be-predicted sequence is determined to include the IRES to separately find a longest common substring of each sample sequence and the to-be-predicted sequence.

In some embodiments, according to the Levenshtein distance-based IRES screening method in the disclosure, the method further includes the following steps of: predicting a secondary structure of the to-be-predicted sequence determined to include the IRES, and determining a position of the IRES in the to-be-predicted sequence in combination with the longest common substring.

In some embodiments, according to the Levenshtein distance-based IRES screening method in the disclosure, predicting the secondary structure of the to-be-predicted sequence determined to include the IRES includes: predicting, by using at least one of RNAfold, Mfold, RNAfoldweerver, and Vienna RNA software, the secondary structure of the to-be-predicted sequence determined to include the IRES.

In some embodiments, according to the Levenshtein distance-based IRES screening method in the disclosure, the secondary structure of the to-be-predicted sequence determined to include the IRES is predicted by using RNAfold software.

In some embodiments, according to the Levenshtein distance-based IRES screening method in the disclosure, the method further includes the following steps: subjecting the to-be-predicted sequence determined to include the IRES to experimental verification to determine the IRES activity of the to-be-predicted sequence.

In some embodiments, according to the Levenshtein distance-based IRES screening method in the disclosure, the experimental verification include the steps of:
constructing a circular nucleic acid molecule by using the to-be-predicted sequence determined to include the IRES, where in the circular nucleic acid molecule, the to-be-predicted sequence is operably linked to a nucleotide sequence encoding a fluorescent protein; and
obtaining a fluorescence signal released by the circular nucleic acid molecule, and determining the IRES activity of the to-be-predicted sequence based on the fluorescence signal.

According to a second aspect, the disclosure provides a polynucleotide, where the polynucleotide is selected from at least one of the group consisting of (i) to (iv):
(i) including a nucleotide sequence shown in any one of SEQ ID NOs: 1, 2, 3, 4, 9, 10, 11, 13, 14, 15, 17, 18, 19, 20, 25, 26, 27, 28, 41, 42, 45, 46, 51, 56, 59, 72, 79, 91, 98, 101, 104, 106, 107, 110, 115, 116, 117, 118, 119, 122, 123, 125, 127, 129, 130, 135, 139, 165, 179, 180, 183, 186, 188, 198, 200, 215, 216, 217, 218, 219, 220, 221, 222, 223, 225, 227, 228, 229, 230, 231, 232, 233, 234, 235, 236, 239, 240, 242, 243, 244, 245, 246, 247, 248, 249, 250, 251, 252, 253, 254, 255, 256, 257, 258, 261, 262, 263, 264, 265, 266, 267, 268, 269, 270, 272, 273, 274, 275, 276, 278, 279, 280, 281, 282, 283, 284, 285, 286, 287, 289, 291, 293, 294, 296, 298, 301, 302, 303, 304, 305, 306, 307, 308, 309, 310, 311, 312, 314, 315, 317, 318, 319, 321, 322, 323, 324, 326, 329, 331, 332, 333, 334, 335, 336, 348, 385, 387, 389, 392, 393, 394, 395, 406, 436, 438, 439, 441, 445, 457, 460, 496, 504, 507, 509, 511, 514, and 534;
(ii) a mutant sequence of any one nucleotide sequence shown in (i), where the mutant sequence has a mutant nucleotide at one or more positions of any corresponding nucleotide sequence shown in (i), and the mutant sequence has an activity of initiating translation of a circular nucleic acid molecule;
(iii) a nucleotide sequence that can be reversely complementary to a hybridized sequence of the nucleotide sequence shown in (i) or (ii) under a highly stringent hybridization condition or a very highly stringent hybridization condition and that has an activity of initiating translation of a circular nucleic acid molecule; and
(iv) a nucleotide sequence having at least 70%, optionally at least 80%, preferably at least 90%, more preferably at least 95%, most preferably at least 98% sequence identity with the nucleotide sequence shown in any one of (i) or (ii) and having an activity of initiating translation of a circular nucleic acid molecule.

Preferably, the polynucleotide includes a nucleotide sequence shown in any of the following sequences:
in some embodiments, according to the polynucleotide in the disclosure, the polynucleotide is a polynucleotide including the IRES that is screened by the method according to any one of claims 1 to 9.

In some embodiments, provided is use of the polynucleotide according to the disclosure in at least one of (a₁)-(a₂):
(a₁) initiating translation of a circular nucleic acid molecule, or preparing a product for initiating translation of a circular nucleic acid molecule; and
(a₂) increasing a protein expression level of a circular nucleic acid molecule, or preparing a product for increasing a protein expression level of a circular nucleic acid molecule.

According to a third aspect, the disclosure provides a circular nucleic acid molecule, where the circular nucleic acid molecule includes the polynucleotide according to the second aspect;
preferably, the circular nucleic acid molecule further includes a coding region encoding a polypeptide of interest, and the coding region is operably linked to the polynucleotide; and
optionally, the circular nucleic acid molecule further includes one or more of the following elements: a 5' spacer region, a 3' spacer region, a second exon, and a first exon.

In some embodiments, according to the circular nucleic acid molecule provided by the disclosure, the 5' spacer region includes a sequence shown in any one of (b₁)-(b₂):
(bi) a nucleotide sequence shown in any one of SEQ ID NOs: 549-550; and
(b₂) a sequence having at least 90%, optionally at least 95%, preferably at least 97%, more preferably at least 98%, most preferably at least 99% sequence identity with the nucleotide sequence shown in (bi).

In some embodiments, according to the circular nucleic acid molecule provided by the disclosure, the 3' spacer region includes a sequence shown in any one of (c₁)-(c₂):
(c₁) a nucleotide sequence shown in any one of SEQ ID NOs: 551-553; and
(c₂) a sequence having at least 90%, optionally at least 95%, preferably at least 97%, more preferably at least 98%, most preferably at least 99% sequence identity with the nucleotide sequence shown in (c₁).

In some embodiments, according to the circular nucleic acid molecule provided by the disclosure, the second exon includes a sequence shown in any one of (d₁)-(d₂):
(d₁) a nucleotide sequence shown in SEQ ID NO: 555; and
(d₂) a sequence having at least 90%, optionally at least 95%, preferably at least 97%, more preferably at least 98%, most preferably at least 99% sequence identity with the nucleotide sequence shown in (d₁).

In some embodiments, according to the circular nucleic acid molecule provided by the disclosure, the first exon includes a sequence shown in any one of (e₁)-(e₂):
(ei) a nucleotide sequence shown in SEQ ID NO: 554; and
(ei) a sequence having at least 90%, optionally at least 95%, preferably at least 97%, more preferably at least 98%, most preferably at least 99% sequence identity with the nucleotide sequence shown in (e₁).

According to a fourth aspect, the disclosure provides a cyclization precursor nucleic acid molecule, where the cyclization precursor nucleic acid molecule is cyclized to form the circular nucleic acid molecule according to the third aspect; and
optionally, the cyclization precursor nucleic acid molecule further includes one or more of the following elements:
a 5' homology arm, a 3' intron, a second exon, a 5' spacer region, a coding region, a 3' spacer region, a first exon, a 5' intron and a 3' homology arm.

In some embodiments, according to the cyclization precursor nucleic acid molecule provided by the disclosure, the 5' homology arm includes a sequence shown in any one of (g₁)-(g₂):
(g₁) a nucleotide sequence shown in any one of SEQ ID NOs: 558-559; and
(g₂) a sequence having at least 90%, optionally at least 95%, preferably at least 97%, more preferably at least 98%, most preferably at least 99% sequence identity with the nucleotide sequence shown in (g₁).

In some embodiments, according to the cyclization precursor nucleic acid molecule provided by the disclosure, the 3' homology arm includes a sequence shown in any one of (h₁)-(h₂):
(hi) a nucleotide sequence shown in any one of SEQ ID NOs: 560-561; and
(hz) a sequence having at least 90%, optionally at least 95%, preferably at least 97%, more preferably at least 98%, most preferably at least 99% sequence identity with the nucleotide sequence shown in (h₁).

In some embodiments, according to the cyclization precursor nucleic acid molecule provided by the disclosure, the 5' intron includes a sequence shown in any one of (j₁)-(j₂):
(j₁) a nucleotide sequence shown in SEQ ID NO: 556; and
(j₂) a sequence having at least 90%, optionally at least 95%, preferably at least 97%, more preferably at least 98%, most preferably at least 99% sequence identity with the nucleotide sequence shown in (j₁).

In some embodiments, according to the cyclization precursor nucleic acid molecule provided by the disclosure, the 3' intron includes a sequence shown in any one of (k₁)-(k₂):
(k₁) a nucleotide sequence shown in SEQ ID NO: 557; and
(k₂) a sequence having at least 90%, optionally at least 95%, preferably at least 97%, more preferably at least 98%, most preferably at least 99% sequence identity with the nucleotide sequence shown in (k₁).

According to a fifth aspect, the disclosure provides a recombinant nucleic acid molecule, where the recombinant nucleic acid molecule is selected from any one of (f₁)-(f₂):
(f₁) including the polynucleotide according to the second aspect; and
(f₂) transcription to form the cyclization precursor nucleic acid molecule according to the fourth aspect.

According to a sixth aspect, the disclosure provides a recombinant expression vector, where the recombinant expression vector includes the recombinant nucleic acid molecule according to the fifth aspect.

According to a seventh aspect, the disclosure provides a recombinant host cell, where the recombinant host cell includes the polynucleotide according to the second aspect, the circular nucleic acid molecule according to the third aspect, the cyclization precursor nucleic acid molecule according to the fourth aspect, the recombinant nucleic acid molecule according to the fifth aspect, or the recombinant expression vector according to the sixth aspect.

According to an eighth aspect, the disclosure provides a method for preparing a circular nucleic acid molecule with an improved protein expression level, where the method includes a step of operably linking the polynucleotide according to the second aspect to a coding region of the circular nucleic acid molecule.

According to a ninth aspect, the disclosure provides use of the circular nucleic acid molecule according to the third aspect, the cyclization precursor nucleic acid molecule according to the fourth aspect, the recombinant nucleic acid molecule according to the fifth aspect, or the recombinant expression vector according to the sixth aspect in at least one of (g₁) to (g₃):
(g₁) expressing a protein, or preparing a product for expressing a protein;
(gz) expressing a polypeptide, or preparing a product for expressing a polypeptide; and
(g₃) serving as or preparing a nucleic acid vaccine;
optionally, the protein or the polypeptide is one or more selected from: an antigen, an antibody, an antigen-binding fragment, a channel protein, a receptor, a cytokine, and an immune checkpoint inhibitor.

### Effects of the Present Invention

In some embodiments, through the Levenshtein distance-based IRES screening method provided by the disclosure, whether there is the IRES in the to-be-predicted sequence can be efficiently and accurately determined. If there is the IRES in the to-be-predicted sequence, a position of the IRES can also be further predicted and determined by further predicting the secondary structure of the to-be-predicted sequence in combination with the longest common substring of the to-be-predicted sequence and the sample sequence, so as to screen out a possible IRES core sequence from the sequences, which provides a technical support for screening of highly active IRESs, facilitates discovery of a new IRES sequence, and helps a researcher to selectively perform experimental verification on a RNA sequence with a higher probability of the presence of an IRES sequence, thereby improving the efficiency of experimental verification and saving ineffective time and costs.

In some embodiments, the polynucleotide shown in any sequence of SEQ ID NOs: 1 to 548 is screened by the method provided by the disclosure. In the disclosure, through experimental verification, it is found that the polynucleotide shown in any sequence of SEQ ID NOs: 1 to 548 has the activity of initiating translation of the circular nucleic acid molecule, which indicates that the screening method provided in the disclosure has an advantage of high accuracy.

In some embodiments, in the disclosure, through comparison, it is found that the polynucleotide including any nucleotide sequence shown in (i) is screened according to the method of the present disclosure, the IRES activity of the polynucleotide exceeds that of a CVB3 IRES element with high translation initiation activity that has been found so far, which can significantly increase the protein expression level of the circular nucleic acid molecule, thereby providing abundant translation initiation elements for application of the circular nucleic acid molecule in preparing a protein, serving as a vaccine, producing a therapeutic protein, or serving as a means of gene therapy, etc.

In some embodiments, the disclosure provides the circular nucleic acid molecule, including the polynucleotide that includes the nucleotide sequence shown in (i), which can achieve a high expression level of a polypeptide of interest and a protein of interest, thereby further expanding the application of the circular nucleic acid molecule in the fields of protein production, prevention or treatment of clinical diseases, etc.

In some embodiments, in the disclosure, the polynucleotide shown in any sequence in (i) is operably linked to the coding region of the circular nucleic acid molecule, providing a good basis for efficient expression of the protein of interest by the circular nucleic acid molecule.

### BRIEF DESCRIPTION OF THE DRAWINGS

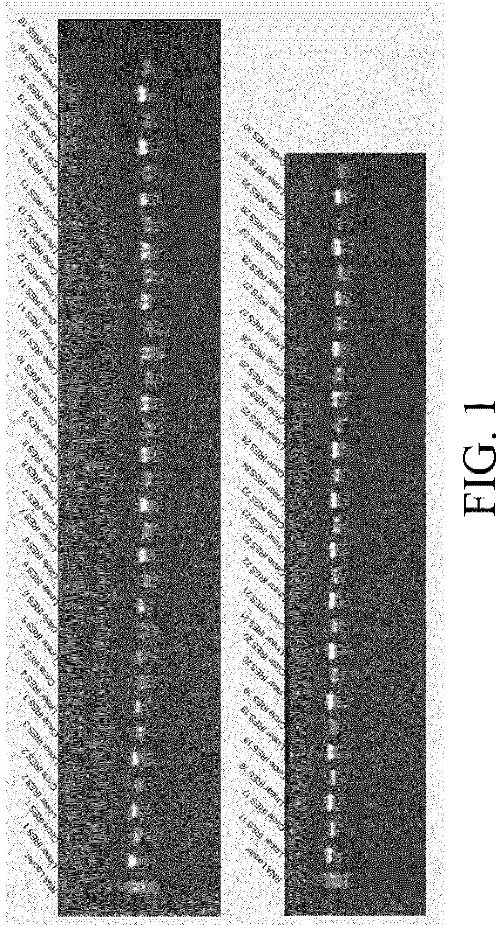

FIG 1 shows a diagram of agarose gel electrophoresis of some linear mRNA molecules and circular mRNA molecules prepared in Example 2, where bands indicated by linear IRESs 1 to 30 in the figure sequentially represent electrophoresis bands of the linear mRNA molecules including polynucleotide sequences shown in SEQ ID NOs: 1 to 30, and bands indicated by circle IRESs 1 to 30 in the figure sequentially represent electrophoresis bands of the circular mRNA molecules including polynucleotide sequences shown in SEQ ID NOs: 1 to 30;

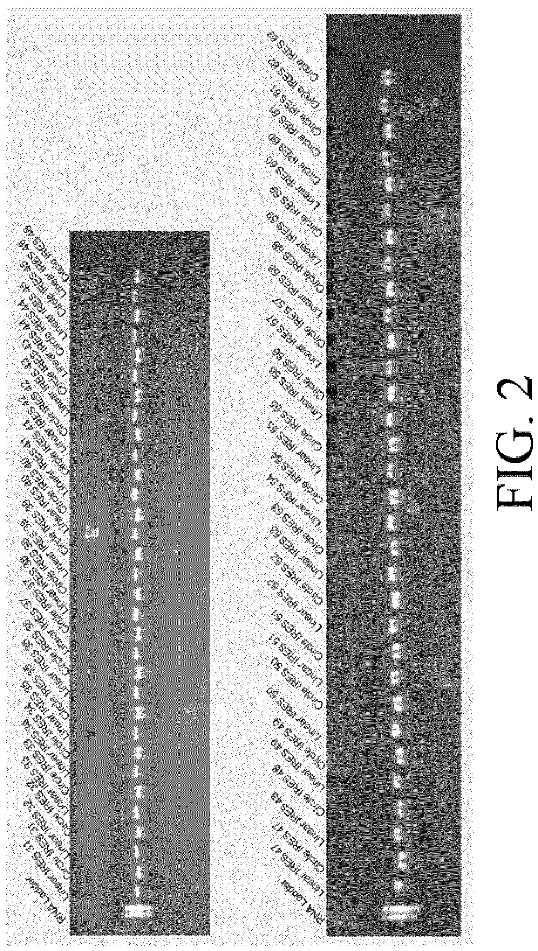

FIG. 2 shows a diagram of agarose gel electrophoresis of some linear mRNA molecules and circular mRNA molecules prepared in Example 2, where bands indicated by linear IRESs 31 to 62 in the figure sequentially represent electrophoresis bands of the linear mRNA molecules including polynucleotide sequences shown in SEQ ID NOs: 31 to 62, and bands indicated by circle IRESs 31 to 62 in the figure sequentially represent electrophoresis bands of the circular mRNA molecules including polynucleotide sequences shown in SEQ ID NOs: 31 to 62;

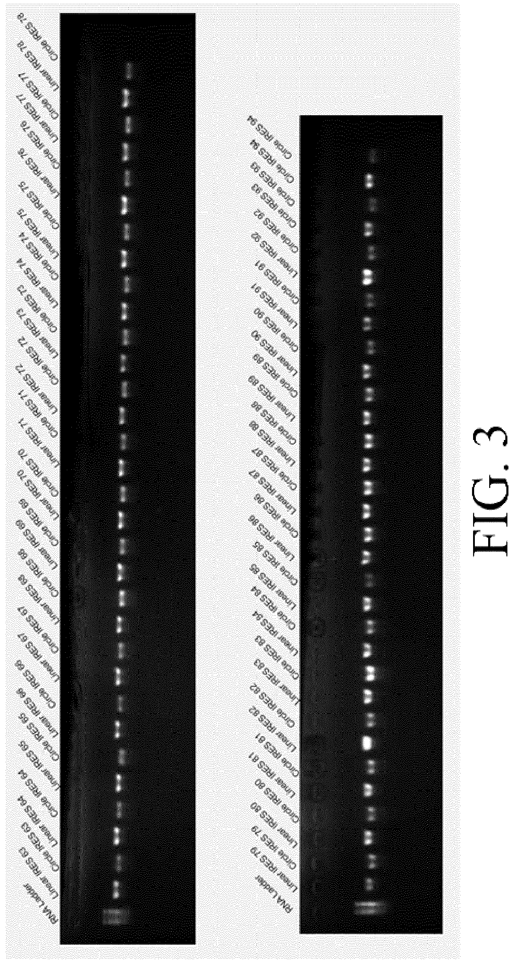

FIG. 3 shows a diagram of agarose gel electrophoresis of some linear mRNA molecules and circular mRNA molecules prepared in Example 2, where bands indicated by linear IRESs 63 to 94 in the figure sequentially represent electrophoresis bands of the linear mRNA molecules including polynucleotide sequences shown in SEQ ID NOs: 63 to 94, and bands indicated by circle IRESs 63 to 94 in the figure sequentially represent electrophoresis bands of the circular mRNA molecules including polynucleotide sequences shown in SEQ ID NOs: 63 to 94;

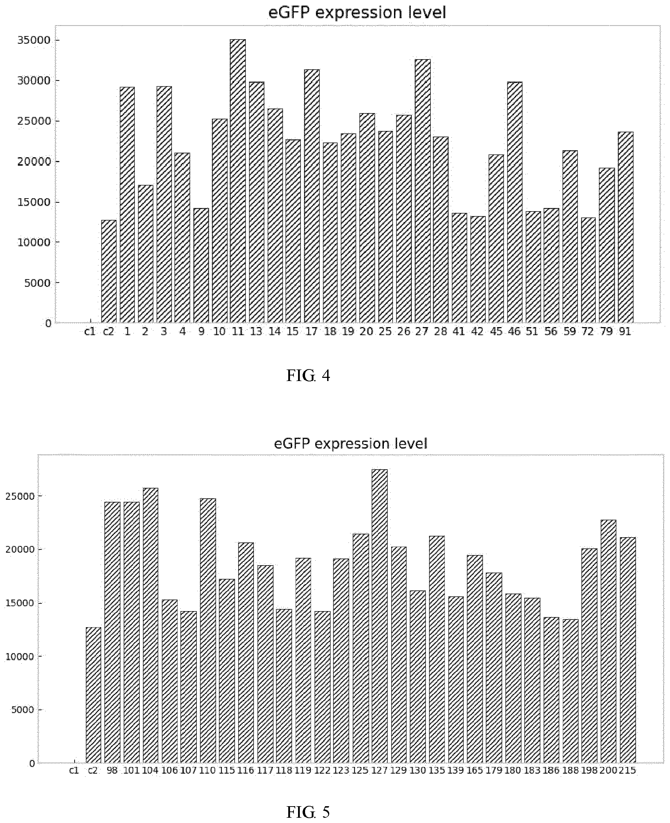

FIG. 4 shows a test result of a fluorescent protein expressed by a circular mRNA molecule constructed by using a polynucleotide obtained by the IRES screening method in the disclosure, where a bar graph in the figure shows a control 1, a control 2, and circular mRNA molecules including nucleotide sequences shown in SEQ ID NOs: 1, 2, 3, 4, 9, 10, 11, 13, 14, 15, 17, 18, 19, 20, 25, 26, 27, 28, 41, 42, 45, 46, 51, 56, 59, 72, 79, and 91 from left to right;
FIG. 5 shows a test result of a fluorescent protein expressed by a circular mRNA molecule constructed by using a polynucleotide obtained by the IRES screening method in the disclosure, where a bar graph in the figure shows a control 1, a control 2, and circular mRNA molecules including nucleotide sequences shown in SEQ ID NOs: 98, 101, 104, 106, 107, 110, 115, 116, 117, 118, 119, 122, 123, 125, 127, 129, 130, 135, 139, 165, 179, 180, 183, 186, 188, 198, 200, and 215 from left to right;

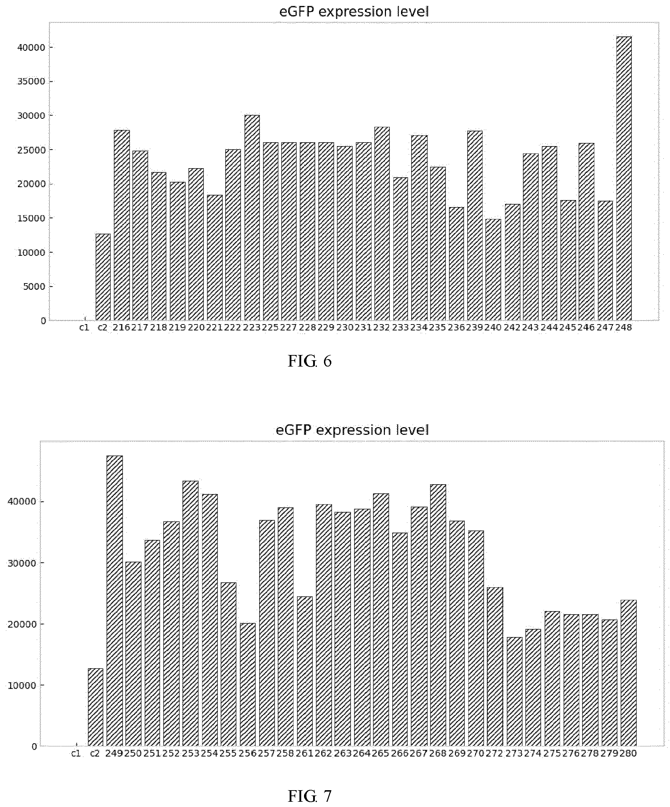

FIG. 6 shows a test result of a fluorescent protein expressed by a circular mRNA molecule constructed by using a polynucleotide obtained by the IRES screening method in the disclosure, where a bar graph in the figure shows a control 1, a control 2, and circular mRNA molecules including nucleotide sequences shown in SEQ ID NOs: 216, 217, 218, 219, 220, 221, 222, 223, 225, 227, 228, 229, 230, 231, 232, 233, 234, 235, 236, 239, 240, 242, 243, 244, 245, 246, 247, and 248 from left to right;
FIG. 7 shows a test result of a fluorescent protein expressed by a circular mRNA molecule constructed by using a polynucleotide obtained by the IRES screening method in the disclosure, where a bar graph in the figure shows a control 1, a control 2, and circular mRNA molecules including nucleotide sequences shown in SEQ ID NOs: 249, 250, 251, 252, 253, 254, 255, 256, 257, 258, 261, 262, 263, 264, 265, 266, 267, 268, 269, 270, 272, 273, 274, 275, 276, 278, 279, and 280 from left to right;

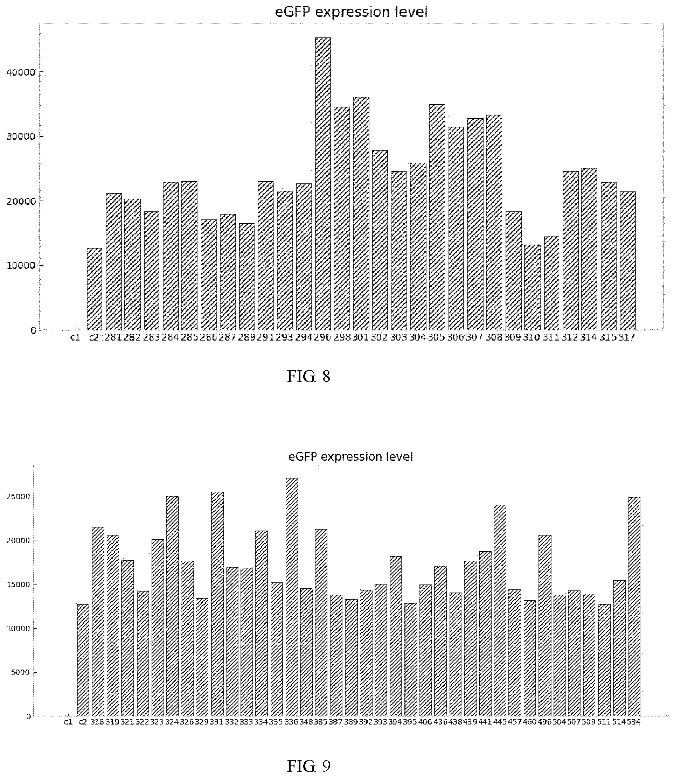

FIG. 8 shows a test result of a fluorescent protein expressed by a circular mRNA molecule constructed by using a polynucleotide obtained by the IRES screening method in the disclosure, where a bar graph in the figure shows a control 1, a control 2, and circular mRNA molecules including nucleotide sequences shown in SEQ ID NOs: 281, 282, 283, 284, 285, 286, 287, 289, 291, 293, 294, 296, 298, 301, 302, 303, 304, 305, 306, 307, 308, 309, 310, 311, 312, 314, 315, and 317 from left to right;
FIG. 9 shows a test result of a fluorescent protein expressed by a circular mRNA molecule constructed by using a polynucleotide obtained by the IRES screening method in the disclosure, where a bar graph in the figure shows a control 1, a control 2, and circular mRNA molecules including nucleotide sequences shown in SEQ ID NOs: 318, 319, 321, 322, 323, 324, 326, 329, 331, 332, 333, 334, 335, 336, 348, 385, 387, 389, 392, 393, 394, 395, 406, 436, 438, 439, 441, 445, 457, 460, 496, 504, 507, 509, 511, 514, and 534 from left to right;

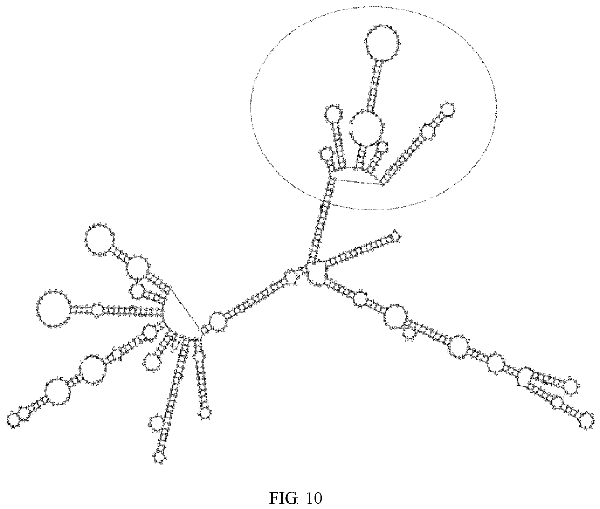

FIG. 10 shows a diagram of a secondary structure of a human poliovirus 1 strain Mahoney_CDC 5'UTR sequence predicted in the disclosure and a position of an IRES; and

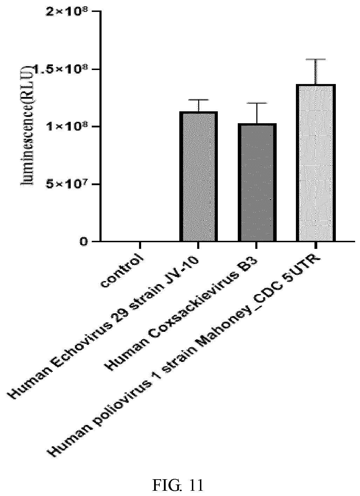

FIG. 11 shows a diagram of test results of luciferase protein expression in a human poliovirus 1 strain Mahoney _CDC 5'UTR group, a human echovirus 29 strain JV-10 group and a human coxsackievirus B3 group.

### DETAILED DESCRIPTION

### Definitions

When used in combination with the term "include" in the claims and/or description, the word "a" or "an" may refer to "one", but may also refer to "one or more", "at least one" and "one or more than one".

As used in the claims and deccription, the word "include", "have", "comprise" or "contain" is meant to be inclusive or open-ended without exclusion of additional unrecited elements or method steps.

Throughout this application document, the term "about" means that one value includes a standard deviation of an error of a device or method used for measuring the value.

Although the disclosed content supports a definition of the term "or" only as a substitute and "and/or", the term "or" in the claims refers to "and/or" unless it is explicitly stated that it is only the substitute or substitutes are mutually exclusive.

The term "one-hot encoding", also known as one-bit valid encoding, mainly means encoding N states by using an N-bit state register, where each state has its own register bit, and only one bit is valid at any time. The one-hot encoding is a representation of a categorical variable as a binary vector. First, a categorical value needs to be mapped to an integer value. Then, each integer value is expressed as a binary vector, which is zero-valued except for an index of an integer, which is denoted as 1.

A term "sample sequence traversing" indicates that sample sequences are objects (or elements) arranged into a column, and each element is either before or after other elements. A sequence between elements is very important. The sample sequence traversing means accessing each element in a sample sequence sequentially along a certain search route once and only once. An operation for accessing the element depends on a specific application problem. Sequence traversing is often used for tree search and graph search of a data structure.

The term "Levenshtein distance" is a measure of a distance between two string sequences. Formally speaking, a Levenshtein distance of two strings is the minimum number of single character editing (for example, deleting, inserting, and substituting) required to transform one string into another string. The Levenshtein distance is also known as an edit distance. Although the Levenshtein distance is only a type of edit distance, the Levenshtein distance is closely related to pairwise string alignment. In mathematics, the Levenshtein distance between two strings a and b satisfy that levab(i, j) = max(i, j), where if min(i, j) = 0, levab(i, j) = min(levab(i - 1, j) + 1, levab(i, j-1) + 1, and levab(i - 1, j - 1) + 1) (ai != bj), where ai != bj is an indicator function. When ai != bj, a value is 1, otherwise, a value is 0. It should be noted that in the minimum item, a first part corresponds to a deletion operation (from a to b), a second part corresponds to an insertion operation, and a third part corresponds to a substitution operation.

The term "maximum common substring" is to find a longest substring of two or more known strings. A difference between a longest common substring and a longest common subsequence is that the subsequences do not have to be continuous, but the substrings must be continuous.

The terms "polypeptide," "peptide," and "protein" are used interchangeably herein and are amino acid polymers of any length. The polymer can be linear or branched, can contain modified amino acids, and can be interrupted by non-amino acids. The term also includes amino acid polymers that have been subjected to modification (for example, disulfide bond formation, glycosylation, lipidation, acetylation, phosphorylation, or any other treatment, such as conjugation with a labeling component).

The term "polynucleotide" or "nucleic acid molecule" refers to a polymer consisting of nucleotides. The polynucleotide may be in a form of an individual fragment or a component of a larger nucleotide sequence structure, derived from nucleotide sequences that have been isolated at least once in quantity or concentration, and sequences and their component nucleotide sequences can be identified, manipulated, and recovered by a standard molecular biological method (for example, by using a cloning vector). When one nucleotide sequence is expressed by one DNA sequence (namely, A, T, G, C), this also indicates inclusion of one RNA sequence (namely, A, U, G, C) where "U" substitutes for "T". In other words, "polynucleotide" refers to a nucleotide polymer removed from other nucleotides (the individual fragment or entire fragment), or may be a component or constituent of the larger nucleotide structure, such as an expression vector or a polycistronic sequence. The polynucleotides include DNA, RNA and cDNA sequences.

The term "circular nucleic acid molecule" refers to a nucleic acid molecule in a closed ring. In some specific embodiments, the circular nucleic acid molecule is a circular RNA molecule. More specifically, the circular nucleic acid molecule is a circular mRNA molecule.

In some embodiments, the circular RNA molecule in the disclosure is formed by linking a 5' end of the upstream of a linear RNA molecule to a 3' end of the downstream of the linear RNA molecule to form a circular form. The circular RNA molecule in the disclosure is formed by subjecting a cyclization precursor RNA molecule to cleavage and a cyclization reaction to form a circular form.

The term "linear RNA" refers to an RNA precursor that can be cyclized to form circular RNA, which is usually transcribed from a linear DNA molecule.

The term "linear RNA" refers to RNA with a translation function including a 5' cap structure, a 3' polyadenosine tail (PolyA tail), a 5' untranslational region (5' UTR), a 3' untranslational region (3' UTR), an open reading frame (ORF), and the like.

The term "translation initiation element" refers to any sequence element capable of recruiting ribosomes and initiating a translation process of an RNA molecule. For example, the translation initiation element is an IRES element, an m⁶A modified sequence, a rolling circle translation initiation sequence, or the like.

The term "IRES" is also known as an internal ribosome entry site, and the "internal ribosome entry site" (IRES) belongs to a translation control sequence, is usually located at a 5' end of a gene of interest, and enables translation of RNA in a cap-independent manner. A transcribed IRES can be directly bound to a ribosomal subunit, so that an mRNA initiation codon is properly oriented in the ribosome for translation. The IRES sequence is usually located in the 5'UTR (just upstream of the initiation codon) of the mRNA. The IRES functionally replaces a requirement for various protein factors that interact with a translation mechanism of eukaryotes.

The term "coding region" refers to a gene sequence capable of transcribing a messenger RNA and finally translating the messenger RNA into a polypeptide or protein of interest.

The term "expression" includes any step involved in production of a polypeptide, which includes, but is not limited to, transcription, post-transcriptional modification, translation, post-translational modification, and secretion.

The terms "sequence identity" and "percent identity" refer to a percentage of same (that is, identical) nucleotides or amino acids of two or more polynucleotides or polypeptides. Sequence identity of two or more polynucleotides or polypeptides can be measured by the following method: aligning nucleotide or amino acid sequences of the polynucleotides or polypeptides, scoring the number of positions containing same nucleotide or amino acid residues in the aligned polynucleotides or polypeptides, and comparing the number of positions containing same nucleotide or amino acid residues in the aligned polynucleotides or polypeptides with the number of positions containing different nucleotide or amino acid residues in the aligned polynucleotides or polypeptides. Polynucleotides can differ at one position, for example, by inclusion of different nucleotides (that is, substitution or mutation) or deletion of nucleotides (that is, insertion of a nucleotide in one or two polynucleotides or deletion of nucleotides). Polypeptides can differ at one position, for example, by inclusion of different amino acids (that is, substitution or mutation) or deletion of amino acids (that is, insertion of an amino acid in one or two polypeptides or deletion of amino acids). The sequence identity can be calculated by dividing the number of the positions containing same nucleotide or amino acid residues by a total number of nucleotide or amino acid residues in the polynucleotides or polypeptides. For example, the percent identity can be calculated by dividing the number of the positions containing same nucleotide or amino acid residues by a total number of nucleotide or amino acid residues in the polynucleotides or polypeptides, and multiplying by 100.

For example, when compared and aligned with maximum correspondence by using a sequence comparison algorithm or measuring via visual inspection, two or more sequences or subsequences have at least 80%, 81%, 82%, 83%, 84%, 85%, 86%, 87%, 88%, 89%, 90%, 91%, 92%, 93%, 94%, 95%, 96%, 97%, 98% or 99% "sequence identity" or "percent identity" of nucleotides. In some embodiments, overall lengths of sequences in any one or two compared biopolymers (for example, polynucleotides) are substantially identical.

The term "recombinant nucleic acid molecule" refers to a polynucleotide having sequences which are not linked together in nature. A recombinant polynucleotide can be included in a proper vector, and the vector can be used for transformation into a proper host cell. The polynucleotide is then expressed in a recombinant host cell to produce, for example, a "recombinant polypeptide", a "recombinant protein", a "fusion protein", and the like.

The term "recombinant expression vector" refers to a DNA structure for expressing, for example, a polynucleotide encoding a required polypeptide. The recombinant expression vector may include: for example, (i) a set of genetic elements having a regulatory effect on gene expression, such as a promoter and an enhancer; (ii) a structure or coding sequence capable of being transcribed into mRNA and translated into protein; and (iii) appropriate transcriptional subunits of transcription and translation initiation and termination sequences. The recombinant expression vector is constructed in any appropriate method. A nature of the vector is not critical and any vector including a plasmid, a virus, a phage, and a transposon can be used. Possible vectors used in the disclosure include, but are not limited to, chromosomal, non-chromosomal, and synthetic DNA sequences, such as a viral plasmid, a bacterial plasmid, a phage DNA, a yeast plasmid, and a vector derived from a combination of plasmid and phage DNA, such as DNAs from viruses such as lentivirus, retrovirus, vaccinia, adenovirus, fowlpox, baculovirus, SV40, and pseudorabies.

The term "host cell" refers to a cell into which an exogenous polynucleotide has been introduced, and includes a progeny of such cell. Host cells include "transformants" and "transformed cells," namely, primary transformed cells and progenies derived therefrom. The host cell is any type of cellular system that can be used to produce an antibody molecule in the present invention, including a eukaryotic cell such as a mammalian cell, an insect cell, and a yeast cell; and a prokaryotic cell such as an Escherichia coli cell. The host cells include cultured cells, and also include cells within transgenic animals, transgenic plants, or cultured plant or animal tissue. The term "recombinant host cell" includes a host cell that differs from a parental cell after introduction of a circular nucleic acid molecule, a cyclization precursor nucleic acid molecule, a recombinant nucleic acid molecule or a recombinant expression vector, and the recombinant host cell is obtained specifically via transformation. The host cell in the disclosure may be a prokaryotic cell or a eukaryotic cell, as long as the host cell is a cell into which the circular nucleic acid molecule, the cyclization precursor nucleic acid molecule, the recombinant nucleic acid molecule, or the recombinant expression vector in the disclosure can be introduced.

The term "highly stringent condition" means subjecting probes of at least 100 nucleotides in length to prehybridization and hybridization treatments for 12 to 24 hours at 42°C in 5X SSPE (saline sodium phosphate EDTA), 0.3% SDS, 200 µg/mL sheared and denatured salmon sperm DNA and 50% formamide according to a standard Southern blotting procedure for the DNA, and finally washing a carrier material with 2X SSC and 0.2% SDS at 65°C for three times, each washing being carried out for 15 minutes.

As used in the disclosure, the term "very highly stringent condition" means subjecting probes of at least 100 nucleotides in length to prehybridization and hybridization for 12 to 24 hours at 42°C in 5X SSPE (saline sodium phosphate EDTA), 0.3% SDS, 200 µg/mL sheared and denatured salmon sperm DNA and 50% formamide according to a standard Southern blotting procedure for the DNA, and finally washing a carrier material with 2X SSC and 0.2% SDS at 70°C for three times, each washing being carried out for 15 minutes.

Unless otherwise defined or clearly indicated in this context, all technical and scientific terms in the disclosure have the same meaning as commonly understood by a person of ordinary skill in the art to which the disclosure belongs.

### Technical solution

In the technical solution in the disclosure, numbers in nucleotide and amino acid sequence listings in the description represent the following meanings:
Sequences shown in SEQ ID Nos: 1 to 548, and 562 to 564 are polynucleotide sequences having an activity of initiating translation of circular nucleic acid molecules;
A sequence shown in a SEQ ID NO: 549 is a nucleotide sequence of a 5' spacer sequence 1;
A sequence shown in SEQ ID NO: 550 is a nucleotide sequence of a 5' spacer sequence 2;
A sequence shown in SEQ ID NO: 551 is a nucleotide sequence of a 3' spacer sequence 1;
A sequence shown in SEQ ID NO: 552 is a nucleotide sequence of a 3' spacer sequence 2;
A sequence shown in SEQ ID NO: 553 is a nucleotide sequence of a 3' spacer sequence 3;
A sequence shown in SEQ ID NO: 554 is a nucleotide sequence of an exon element 1 (E1) of a class I PIE system;
A sequence shown in SEQ ID NO: 555 is a nucleotide sequence of an exon element 2 (E2) of a class I PIE system;
A sequence shown in a SEQ ID NO: 556 is a nucleotide sequence of a 5' intron of a class I PIE system;
A sequence shown in SEQ ID NO: 557 is a nucleotide sequence of a 3' intron of a class I PIE system;
A sequence shown in SEQ ID NO: 558 is a nucleotide sequence of a 5' homology arm sequence 1 (H1);
A sequence shown in SEQ ID NO: 559 is a nucleotide sequence of a 5' homology arm sequence 2 (H2);
A sequence shown in SEQ ID NO: 560 is a nucleotide sequence of a 3' homology arm sequence 1; and
A sequence shown in SEQ ID NO: 561 is a nucleotide sequence of a 3' homology arm sequence 2.

### Levenshtein distance-based IRES screening method

The Levenshtein distance-based IRES screening method in the disclosure includes the following steps:
(1) selecting n sequences including an IRES as sample sequences, where n≥1 and n is a natural number;
(2) subjecting the sample sequences and to-be-predicted sequences to one-hot encoding respectively, where categorical variables are A, T, C, and G;
(3) traversing the sample sequences, and calculating a Levenshtein distance between each sample sequence and the to-be-predicted sequence;
(4) calculating an average of Levenshtein distances between all sample sequences and the to-be-predicted sequences; and
(5) determining, based on the average, whether the to-be-predicted sequences include the IRES.

According to the screening method provided by the disclosure, the Levenshtein distance is used for the first time to screen and determine IRESs for a large number of to-be-predicted sequence samples, which helps the researchers to selectively perform experimental verification on the to-be-predicted sequence samples with a high probability of the presence of the IRES, thereby effectively reducing time and costs for IRES sequence screening. Compared with an existing IRES prediction method, the screening method in the disclosure has advantages of accurate results and high efficiency.

In some embodiments, in the step (5), if the average is not less than a set prediction threshold, it is determined that the to-be-predicted sequence includes the IRES, otherwise it is determined that the to-be-predicted sequence includes no IRES.

In some specific embodiments, the prediction threshold is not less than 0.5. When the prediction threshold is not less than 0.5, there is a high probability that the to-be-predicted sequence includes the IRES. In some preferable embodiments, the prediction threshold is 0.75. When the prediction threshold is 0.75, the to-be-predicted sequences generally include the IRES.

In some specific embodiments, a Levenshtein distance calculation method is as follows: a Levenshtein distance between two strings a and b satisfy that levab(i, j) = max(i, j), where if min(i, j) = 0, levab(i, j) = min(levab(i - 1, j) + 1, levab(i, j - 1) + 1, and levab(i - 1, j - 1) + 1) (ai != bj), where ai != bj is an indicator function. When ai != bj, a value is 1, otherwise, a value is 0. It should be noted that in the minimum item, a first part corresponds to a deletion operation (from a to b), a second part corresponds to an insertion operation, and a third part corresponds to a substitution operation.

In some embodiments, the method further includes the following steps: predicting a secondary structure of the to-be-predicted sequence determined to include the IRES, and determining a position of the IRES in the to-be-predicted sequence in combination with the longest common substring.

Further, predicting the secondary structure of the to-be-predicted sequence determined to include the IRES includes: predicting, by using at least one of RNAfold, Mfold, RNAfoldweerver, and Vienna RNA software, the secondary structure of the to-be-predicted sequence determined to include the IRES.

In combination with IRES analysis software such as RNAfold, the position of IRES in the to-be-predicted sequence containing IRES can be further analyzed and located, which facilitates the discovery of new IRES sequences.

In some embodiments, the method further includes the following step of: subjecting the to-be-predicted sequence determined to include the IRES to experimental verification to determine an IRES activity of the to-be-predicted sequence.

In some embodiments, the experimental verification includes the steps of constructing a circular nucleic acid molecule by using the to-be-predicted sequence determined to include the IRES, where in the circular nucleic acid molecule, the to-be-predicted sequence is operably linked to a nucleotide sequence encoding a fluorescent protein; and
obtaining a fluorescence signal released by the circular nucleic acid molecule, and determining the IRES activity of the to-be-predicted sequence based on the fluorescence signal.

In some specific embodiments, in the disclosure, by taking the condition that disclosed human poliovirus 1 strain Mahoney_CDC 5' UTR (a sequence shown in SEQ ID NO: 564) with the IRES activity is used as a to-be-predicted sequence as an example, a process of determining, by the method in the disclosure, whether the sequence shown in SEQ ID NO: 564 contains the IRES is as follows:
(1) selection of a sample sequence: a highly active human Coxsackievirus B3 (CVB3) virus IRES sequence (SEQ ID NO: 562) and a highly active human Echovirus 29 strain JV-10 (E29) virus IRES sequence (SEQ ID NO: 563) that have been experimentally verified are selected as sample sequences;
(2) one-hot encoding: as shown in Tables 1-3 below, to-be-encoded objects are determined as the sample sequence and the to-be-predicted sequence, where the categorical variables are A, T, C, and G; and each sample has 4 features, and the features are converted into binary vectors for representation, thereby converting sequence letter information into digital information;

**Table 1 (SEQ ID NO: 562)**

| | T | T | A | A | A | A | C | A | G | ... | T | A | C | A | G | C | A | A | A |
|---|---|---|---|---|---|---|---|---|---|---|---|---|---|---|---|---|---|---|---|
| A | 0 | 0 | 1 | 1 | 1 | 1 | 0 | 1 | 0 | ... | 0 | 1 | 0 | 1 | 0 | 0 | 1 | 1 | 1 |
| T | 1 | 1 | 0 | 0 | 0 | 0 | 0 | 0 | 0 | ... | 1 | 0 | 0 | 0 | 0 | 0 | 0 | 0 | 0 |
| C | 0 | 0 | 0 | 0 | 0 | 0 | 1 | 0 | 0 | ... | 0 | 0 | 1 | 0 | 0 | 1 | 0 | 0 | 0 |
| G | 0 | 0 | 0 | 0 | 0 | 0 | 0 | 0 | 1 | ... | 0 | 0 | 0 | 0 | 1 | 0 | 0 | 0 | 0 |

**Table 2 SEQ ID NO: 563)**

| | T | T | A | A | A | A | C | A | G | ... | C | A | C | C | G | C | A | A | A |
|---|---|---|---|---|---|---|---|---|---|---|---|---|---|---|---|---|---|---|---|
| A | 0 | 0 | 1 | 1 | 1 | 1 | 0 | 1 | 0 | ... | 0 | 1 | 0 | 0 | 0 | 0 | 1 | 1 | 1 |
| T | 1 | 1 | 0 | 0 | 0 | 0 | 0 | 0 | 0 | ... | 0 | 0 | 0 | 0 | 0 | 0 | 0 | 0 | 0 |
| C | 0 | 0 | 0 | 0 | 0 | 0 | 1 | 0 | 0 | ... | 1 | 0 | 1 | 1 | 0 | 1 | 0 | 0 | 0 |
| G | 0 | 0 | 0 | 0 | 0 | 0 | 0 | 0 | 1 | ... | 0 | 0 | 0 | 0 | 1 | 0 | 0 | 0 | 0 |

**Table 3 (SEQ ID NO: 564)**

| | T | T | A | A | A | A | C | A | G | ... | T | G | T | A | T | C | A | T | A |
|---|---|---|---|---|---|---|---|---|---|---|---|---|---|---|---|---|---|---|---|
| A | 0 | 0 | 1 | 1 | 1 | 1 | 0 | 1 | 0 | ... | 0 | 0 | 0 | 1 | 0 | 0 | 1 | 0 | 1 |
| T | 1 | 1 | 0 | 0 | 0 | 0 | 0 | 0 | 0 | ... | 1 | 0 | 1 | 0 | 1 | 0 | 0 | 1 | 0 |
| C | 0 | 0 | 0 | 0 | 0 | 0 | 1 | 0 | 0 | ... | 0 | 0 | 0 | 0 | 0 | 1 | 0 | 0 | 0 |
| G | 0 | 0 | 0 | 0 | 0 | 0 | 0 | 0 | 1 | ... | 0 | 1 | 0 | 0 | 0 | 0 | 0 | 0 | 0 |

(3) the sample sequences are traversed, and a Levenshtein distance between each sample sequence and the to-be-predicted sequence is calculated: wherein a representes the sample sequence, b representes the to-be-predicted sequence, i and j respectively represent a row and a column in Tables 1-3, and based on a calculation formula of the Levenshtein distance, a Levenshtein distance between the human poliovirus 1 strain Mahoney_CDC 5'UTR sequence and the human Coxsackievirus B3 (CVB3) virus IRES sequence is calculated to be 0.79028, and a Levenshtein distance between the human poliovirus 1 strain Mahoney _CDC 5'UTR sequence and the human Echovirus 29 strain JV-10 (E29) virus IRES sequence is calculated to be 0.79380;
(4) a prediction threshold is set to be 0.75, and an average of Levenshtein distances between 2 sample sequences and the to-be-predicted sequence is calculated to be 0.79204, where the average is greater than the prediction threshold of 0.75, and therefore, the to-be-predicted sequence, human poliovirus 1 strain Mahoney_CDC 5'UTR sequence, can be determined as the IRES-containing sequence;
(5) the sample sequences are traversed, and the longest common substrings of each sample sequence and the to-be-predicted sequence are separately searched, where the longest common substring of the to-be-predicted sequence, the human poliovirus 1 strain Mahoney _CDC 5'UTR sequence, and the sample sequence, the human Coxsackievirus B3 (CVB3) virus IRES sequence, is GCGGAACCGACTACTTTGGGTGTCCGTGTTTC, and the longest common substring of the to-be-predicted sequence, the human poliovirus 1 strain Mahoney _CDC 5'UTR sequence, and the sample sequence, the human Echovirus 29 strain JV-10 (E29) virus IRES sequence, is TCCTCCGGCCCCTGAATGCGGCTAATCCCAAC; and
(6) a secondary structure of the human poliovirus 1 strain Mahoney_CDC 5'UTR sequence is predicted by using RNAfold software, where as shown in FIG. 10, in combination with the longest common substring, it can be predicted that an IRES structure in the human poliovirus 1 strain Mahoney_CDC 5'UTR sequence is within a region marked by an oval circle.

As shown in FIG. 11, luciferase protein expression results reveal that mRNA and protein expression of the human poliovirus 1 strain Mahoney_CDC 5'UTR group is significantly higher than that of the control groups, the human echovirus 29 strain JV-10 group and the human coxsackievirus B3 group. It can thus be seen that the to-be-predicted sequence, the human poliovirus 1 strain Mahoney _CDC 5'UTR sequence, that is determined to include the IRES by the Levenshtein distance-based IRES screening method provided by the disclosure does include the IRES through experimental verification, and can be applied to expression of the circular RNA, and the IRES activity of the human poliovirus 1 strain Mahoney_CDC 5'UTR sequence is significantly higher than that of the sample sequences, the human Coxsackievirus B3 (CVB3) virus IRES sequence and the human echovirus 29 strain JV-10 (E29) virus IRES sequence. Therefore, it is proved that the Levenshtein distance-based IRES prediction method provided by the present invention has high prediction accuracy, and can be used to efficiently and accurately predict whether there is the IRES in the to-be-predicted sequence, and the IRES screened by the IRES prediction method provided by the present invention has higher activity and can be applied to the expression of the circular RNA.

Further, by the foregoing method, 548 nucleotide sequences containing the IRES are found via screening in the disclosure, and during further experimental verification, in the disclosure, it is found that a nucleotide sequence shown in any one of SEQ ID NOs: 1 to 548 has the IRES activity and can initiate the expression of a protein of interest in the circular nucleic acid molecule, indicating that the screening method provided by the disclosure has the advantages of high accuracy and high efficiency.

It should be noted that CVB3 IRES is a currently discovered IRES element having high IRES activity and capable of initiating protein expression of the circular nucleic acid molecule to high extent (Wesselhoeft RA, Kowalski PS, Anderson DG Engineering circular RNA for potent and stable translation in eukaryotic cells. Nat Commun. 2018 Jul 6; 9(1): 2629. doi: 10.1038/s41467-018-05096-6.). In some specific embodiments, in the disclosure, by using the currently discovered CVB3 IRES having high IRES activity as a control, it is found that the polynucleotides of sequences shown below (SEQ ID NOs: 1, 2, 3, 4, 9, 10, 11, 13, 14, 15, 17, 18, 19, 20, 25, 26, 27, 28, 41, 42, 45, 46, 51, 56, 59, 72, 79, 91, 98, 101, 104, 106, 107, 110, 115, 116, 117, 118, 119, 122, 123, 125, 127, 129, 130, 135, 139, 165, 179, 180, 183, 186, 188, 198, 200, 215, 216, 217, 218, 219, 220, 221, 222, 223, 225, 227, 228, 229, 230, 231, 232, 233, 234, 235, 236, 239, 240, 242, 243, 244, 245, 246, 247, 248, 249, 250, 251, 252, 253, 254, 255, 256, 257, 258, 261, 262, 263, 264, 265, 266, 267, 268, 269, 270, 272, 273, 274, 275, 276, 278, 279, 280, 281, 282, 283, 284, 285, 286, 287, 289, 291, 293, 294, 296, 298, 301, 302, 303, 304, 305, 306, 307, 308, 309, 310, 311, 312, 314, 315, 317, 318, 319, 321, 322, 323, 324, 326, 329, 331, 332, 333, 334, 335, 336, 348, 385, 387, 389, 392, 393, 394, 395, 406, 436, 438, 439, 441, 445, 457, 460, 496, 504, 507, 509, 511, 514, and 534) in the disclosure have a higher capability of initiating the protein expression of the circular mRNA molecule compared with CVB3 IRES, indicating that a large number of nucleotide sequences of interest having extremely high IRES activity can be screened by the method in the disclosure, which lays a foundation for improving the level of the protein of interest expressed by the circular nucleic acid molecule.

### Polynucleotide having activity of initiating translation of circular nucleic acid molecule

Currently, although IRES elements capable of initiating a protein translation process have been found in some species (such as viruses), homology of viral IRES sequences of different species is low, and currently there is a lack of definite standards for determining the IRES sequences. Therefore, further research and identification are needed for the IRES sequences having the activity of initiating translation of the circular nucleic acid molecules.

To resolve the foregoing problem, the disclosure provides polynucleotides derived from different types of viruses as follows:
Echovirus E1 (strain Farouk / ATCC VR-1038), Echovirus E2 (strain USA/2013-19511), Echovirus E3 (isolate JSev001), Echovirus E3 (strain 61246-70294), Echovirus E3 (strain 61247-622), Echovirus E3 (strain 61245-2710), Echovirus E3 (strain 63038-1131), Echovirus E3 (strain 63040-70881), Echovirus E3 (isolate HNWY-01), Echovirus E3 (isolate ECHO3_INMI1), Echovirus E3 (isolate Env_2016_Sep_E-3), Echovirus E3 (strain Sakhalin-11.293), Echovirus E3 (strain HAI/2016-23067A), Echovirus E3 (strain HAI/2016-23066), Echovirus E3 (strain HAI/2016-23065A), Echovirus E3 (strain HAI/2016-23061), Echovirus E3 (strain HAI/2016-23056), Echovirus E3 (strain HAI/2016-23051A), Echovirus E3 (strain HAI/2016-23050), Echovirus E3 (isolate 123-R2), Echovirus E3 (strain Sakhalin/10_DU145), Echovirus E3 (strain Sakhalin/10_RD), Echovirus E3 (isolate E3/TO/BR/018), Echovirus E4 (strain 2F5), Echovirus 4 (strain AUS250G), Echovirus E4 (strain Pesacek), Echovirus E5, Echovirus E6, Echovirus 9 (strain Barty), Echovirus 9 (strain Hill), Echovirus E11, Echovirus E12, Echovirus E13 (strain HAI/2017-23078B), Echovirus E13 (strain HAI/2016-23072), Echovirus E13 (strain HAI/2016-23073), Echovirus E13 (strain HAI/2016-23075), Echovirus E13 (strain HAI/2017-23082B), Echovirus E14 (strain RO-81-1-79), Echovirus E14 (isolate ETH_P19/E14_2016), Echovirus E14 (isolate NSW-V04-2012-ECHO14), Echovirus E14 (isolate E14/P843/2013/China), Echovirus E14 (isolate E14/P968/2013/China), Echovirus E15 (strain CH 96-51), Echovirus E16 (isolate ETH_P4/E16_2016), Echovirus E16 (isolate E16/P85/2013/China), Echovirus E16 (strain Harrington), Echovirus 17 (strain CHHE-29), Echovirus E18 (isolate PC06/JS/CHN/2019), Echovirus E18 (strain E18/JXY2-2/2019), Echovirus E18 (isolate QD9/SD/CHN/2019), Echovirus E18 (isolate LJ/0530/2019), Echovirus E18 (strain 12J3), Echovirus E18 (strain USA/2015/CA-RGDS-1049), Echovirus E18 (isolate E18-221/HeB/CHN/2015), Echovirus E18 (strain 12G5), Echovirus E18 (isolate E18-393/HeB/CHN/2015), Echovirus E18 (isolate E18-398/HeB/CHN/2015), Echovirus E18 (isolate E18-HeB15-54462/HeB/CHN/2015), Echovirus E18 (isolate E18-HeB15-54498/HeB/CHN/2015), Echovirus E18 (isolate ETH_P12/E18_2016), Echovirus E18 (isolate NSW-V13A-2008-ECHO18), Echovirus E18 (strain A83/YN/CHN/2016), Echovirus E18 (strain A86/YN/CHN/2016), Echovirus E18 (isolate Jena/ST9524/10), Echovirus E18 (isolate Jena/VI10227/10), Echovirus E18 (isolate Kor05-ECV18-054cn), Echovirus E19 (strain HAI/2016-23039B), Echovirus E19 (strain HAI/2016-23036D), Echovirus E19 (strain HAI/2016-23037D), Echovirus E19 (strain HAI/2016-23037E), Echovirus E19 (strain HAI/2016-23042B), Echovirus E19 (strain HAI/2016-23046B), Echovirus E19 (strain HAI/2016-23047), Echovirus E19 (strain HAI/2016-23054), Echovirus E19 (strain HAI/2016-23052), Echovirus E19 (strain HAI/2016-23053), Echovirus E19 (strain HAI/2016-23062D), Echovirus E19 (strain HAI/2016-23063B), Echovirus E19 (strain HAI/2016-23064B), Echovirus E19 (strain HAI/2016-23067B), Echovirus E19 (strain HAI/2016-23070B), Echovirus E19 (strain HAI/2017-23079), Echovirus E19 (strain HAI/2017-23081A), Echovirus E19 (isolate ETH_P3/E19_2016), Echovirus E19 (strain NGR_2014), Echovirus E19 (isolate PDV_BLR_IN), Echovirus E19 (strain Burke), Echovirus E19 (strain K/542/81), Echovirus E20 (isolate E20/TO/BR/016), Echovirus E20 (strain HAI/2016-23038B), Echovirus E20 (strain HAI/2016-23041B), Echovirus E20 (strain HAI/2016-23085B), Echovirus E20 (strain HAI/2016-23065C), Echovirus E20 (strain HAI/2016-23068B), Echovirus E20 (strain HAI/2016-23069), Echovirus E20 (strain HAI/2017-23080B), Echovirus E20 (strain HAI/2017-23081B), Echovirus E20 (HAI/2016-23077B), Echovirus E20 (strain HAI/2017-23083C), Echovirus E20 (strain KM-EV20-2010), Echovirus E20 (strain JV-1), Echovirus E21 (strain 553/YN/CHN/2013), Echovirus E21 (strain Farina), Echovirus E24 (strain VEN/2018-23086), Echovirus E24 (isolate PZ18G/JS/20120703), Echovirus E24 (strain DeCamp), Echovirus E25 (strain USA/2016-19521), Echovirus E25 (strain USA/2018-23126), Echovirus E25 (strain 10-4339-2), Echovirus E25 (strain USA/CA/RGDS-2017-1010), Echovirus E25 (isolate NSW-V07-2007-ECHO25), Echovirus E25 (isolate NSW-V08-2008-ECHO25), Echovirus E25 (isolate NSW-V09-2008-ECHO25), Echovirus E25 (isolate NSW-V58-2010-ECHO25), Echovirus E25 (strain 61241-70868), Echovirus E25 (strain E25/ZE-wly/Zhejiang/CHN/2005), Echovirus E25 (isolate Jena/AN1380/10), Echovirus E25 (strain XM0297), Echovirus E25 (strain E25/2010/CHN/BJ), Echovirus E25 (isolate E25SD2010CHN), Echovirus E25 (strain HN-2), Echovirus E25 (strain JV-4), Echovirus E26 (strain Coronel), Echovirus E27 (isolate ETH_P8/E27_2016), Echovirus E27 (strain Bacon), Echovirus E29 (strain HAI/2016-23048B), Echovirus E29 (strain JV-10), Echovirus E30 (isolate E30/TO/BR/032), Echovirus E30 (isolate TL12C/NM/CHN/2016), Echovirus E30 (isolate TL7C/NM/CHN/2016), Echovirus E30 (strain USA/2018-23125), Echovirus E30 (Echo30/Hokkaido.JPN/21208/2017), Echovirus E30 (strain USA/2015/CA-RGDS-1046), Echovirus E30 (strain USA/2017/CA-RGDS-1048), Echovirus E30 (isolate B001/USA/2016), Echovirus E30 (strain 16-I10), Echovirus E30 (strain 1-B4-TW), Echovirus E30 (strain 2002-59), Echovirus E30 (strain KM/A363/09), Echovirus E30 (isolate 1-MRS2013), Echovirus E30 (isolate 3-MRS2013), Echovirus E30 (isolate 4-MRS2013), Echovirus E30 (isolate 2012EM161), Echovirus E30 (isolate E30SD2010CHN), Echovirus E30 (isolate ECV30/GX10/05), Echovirus E30 (strain Kor08-ECV30), Echovirus E30 (isolate FDJS03_84), Echovirus 30 (strain Bastianni), Echovirus 31 (strain Caldwell), Echovirus 32 (strain PR-10), Echovirus E33 (strain YNK35/CHN/2013), Echovirus E33 (strain YNA12/CHN/2013), Human poliovirus 1 (isolate CHN-Hainan/93-2), Human poliovirus 1 (isolate RUS39223), Human poliovirus 1 (isolate Pak-1), Human poliovirus 1 (isolate TJK35363 clone 6), Human poliovirus 1 (strain 3788ALB96), Human poliovirus 1 (isolate CHN15115/Xinjiang/CHN/2011), Human poliovirus 1 (isolate 29690_c1), Human poliovirus 1 (strain NIE1018316), Human poliovirus 1 (isolate EGY1218587), Human poliovirus 1 (isolate 558BRA-PE/88), Human poliovirus 2 (isolate Env2008_E2450), Human poliovirus 2 (strain CHA1218985), Human poliovirus 2 (isolate Env2008_E3218), Human poliovirus 2 (strain MAD-2593-11), Human poliovirus 3 (strain PAK1019536), Human poliovirus 3 (isolate Env08_E2886), Human poliovirus 3 (strain SWI10947), Human poliovirus 3 (strain FIN84-2493), Human poliovirus 3 (strain USOL-D-bac), Enterovirus A71 (isolate 2019-EV-A71-R398), Enterovirus A71 (strain USA/2018-23296), Enterovirus A71 (strain 16L), Enterovirus A76 (strain 10-3291-2), Human enterovirus A76 (AY697458), Enterovirus A89 (strain KSYPH-TRMH22F/XJ/CHN/2011), Human enterovirus A89 (AY697459.1), Enterovirus A90 (strain 10-2879-1), Enterovirus A90 (isolate SCH05F/XJ/CHN/2011), Human enterovirus A90 (isolate 01336/SD/CHN/EV90), Human enterovirus A90 (AB192877.1), Human enterovirus A90 (isolate F950027), Human enterovirus 91 (AY697461.1), Human enterovirus A92 (strain RJG7), Simian enterovirus SV19 (strain NOLA-2), Simian enterovirus SV19 (isolate cg4006), Simian enterovirus SV19 (strain M19s (P2)), Simian enterovirus SV43 (strain OM112t (P12)), Simian enterovirus SV46 (isolate cg5400), Simian enterovirus SV46 (strain RNM5), Enterovirus B69 (strain Toluca-1), Enterovirus B69 (isolate 15_491), Enterovirus B73 (isolate 088/SD/CHN/04), Human enterovirus B73 (isolate 2776-82), Human enterovirus 74 (strain Rikaze-136/XZ/CHN/2010), Enterovirus B75 (isolate Y16/XZ/CHN/2007), Enterovirus B75 (isolate 102/SD/CHN/97), Enterovirus B75 (strain USA/OK85-10362), Human enterovirus B77 (strain USA/TX97-10394), Human enterovirus B77 (strain CF496-99), Human enterovirus B79 (strain 17-2255-1_E79), Human enterovirus B79 (AB426610.1), Human enterovirus B79 (strain USA/CA79-10384), Enterovirus B80 (isolate HT-LYKH203F/XJ/CHN/2011), Human enterovirus B80 (isolate HZ01/SD/CHN/2004), Enterovirus B81 (isolate 99279/XZ/CHN/1999), Human enterovirus B81 (strain USA/CA68-10389), Human enterovirus B82 (strain USA/CA64-10390), Human enterovirus B83 (strain USA/CA76-10392), Enterovirus B83 (isolate 99245/XZ/CHN/1999), Enterovirus B83 (isolate AFP341-GD-CHN-2001), Enterovirus B83 (isolate 246/YN/CHN/08), Enterovirus B84 (strain GHA:BAR:TES/2017), Enterovirus B84 (isolate AFP452/GD/CHN/2004), Human enterovirus B84 (isolate CIV2003-10603), Human enterovirus B85 (strain HTPS-MKLH04F/XJ/CHN/2011), Human enterovirus B85 (strain BAN00-10353), Human enterovirus B86 (strain BAN00-10354), Enterovirus B87 (isolate LY02/SD/CHN/2000), Enterovirus B88 (strain 11-4644-1), Human enterovirus B88 (strain BAN01-10398), Enterovirus B93 (isolate 99052/XZ/CHN/1999), Enterovirus B93 (isolate 38-03), Human enterovirus B97 (strain 99188/SD/CHN/1999/EV97), Human enterovirus B97 (strain DT94-0227), Human enterovirus B97 (strain BAN99-10355), Human enterovirus B98 (strain: T92-1499), Human enterovirus B100 (isolate BAN2000-10500), Human enterovirus B101 (strain CIV03-10361), Enterovirus B106 (isolate AKS-AWT-AFP2F/XJ/CHN/2011), Human enterovirus 106 (isolate 148/YN/CHN/12), Enterovirus C96 (strain VEN/2018-23123A), Enterovirus C96 (isolate 127/SD/CHN/1991), Enterovirus C96 (clone V13C), Enterovirus C99 (strain 10L1), Human enterovirus C104 (isolate kvv585-16-TS), Human enterovirus C105 (strain USA/OK/2014-19362), Human enterovirus C116 (strain 126), Enterovirus C117 (strain JX-C117-40-2017), Human enterovirus C118 (isolate CQ5185), Human enterovirus D68 (strain Fermon), Enterovirus D68 (TBp-13-Ph209), Enterovirus D70 (strain JPN/1989-23292), Enterovirus D94 (strain ANG/2010-23293), Human enterovirus D94 (isolate 19/04), Enterovirus D111 (strain ANG/2010-23294), Enterovirus D111 (isolate D111-NGR-KAT-1263), Simian enterovirus J103 (isolate cg8227), Coxsackievirus A2 (isolate HN202009), Coxsackievirus A2 (isolate 16027), Coxsackievirus A2 (isolate CVA2-1388-M14/XY/CHN/2017), Coxsackievirus A2 (isolate CVA2/Shenzhen50/CHN/2012), Coxsackievirus A2 (strain 2260165), Coxsackievirus A4 (strain CA4/JX2204/2014), Coxsackievirus A4 (isolate HK458564/2016), Coxsackievirus A5 (isolate CV-A5-3487-M14-XY-CHN-2017), Coxsackievirus A5 (strain CVA5/13164/HUN/2015), Coxsackievirus A6 (isolate DN1501), Coxsackievirus A6 (strain RYN-A1205), Coxsackievirus A7 (strain MAD-3101-11), Coxsackievirus A8 (isolate 13-467/GS/CHN/2013), Coxsackievirus A8 (isolate C177/CHW/AUS/2017), Coxsackievirus A8 (isolate CV-A8/P82/2013/China), Human coxsackievirus A8 (strain Donovan), Coxsackievirus A10 (isolate TA111R), Coxsackievirus A10 (strain CA10/JX2545/2017), Coxsackievirus A12 (isolate D89), Coxsackievirus A12 (strain QD-LXH535/SD/CHN/2009), Coxsackievirus A14 (strain MAD-72-07), Coxsackievirus A14 (isolate SEN-14-254), Human coxsackievirus A14 (strain G-14), Coxsackievirus A16 (isolate AH17-18/AH/East/CHN/2017-02-12), Coxsackievirus A16 (isolate CV-A16/HVN08.039_HA_GIANGVNM/2008), Coxsackievirus B1 (strain RO-98-1-74), Coxsackievirus B1 (strain CVB1/XM0108), Coxsackievirus B1 (strain B1/Groningen/2011), Coxsackievirus B2 (strain 13-2380-2_B2), Coxsackievirus B2 (strain 14L), Coxsackievirus B2 (strain 08-749-Shimane08-JPN), Coxsackievirus B2 (strain RW41-2/YN/CHN/2012), Coxsackievirus B2 (isolate BCH314), Coxsackievirus B3 (isolate B307), Coxsackievirus B3 (isolate 2001-5), Coxsackievirus B3 (isolate DH09Y/JS/2012), Coxsackievirus B4 (isolate B401), Coxsackievirus B4 (isolate CV-B4/P11/2013/China), Coxsackievirus B4 (isolate Edwards CB4), Coxsackievirus B5 (isolate B501), Coxsackievirus B5 (strain USA/MI/2009-23030), Coxsackievirus B6 (isolate 99148/XZ/CHN/1999), Coxsackievirus B6 (strain LEV15), Coxsackievirus A9 (strain A744/YN/CHN/2009), Coxsackievirus A9 (isolate 2-MRS2013), Coxsackievirus A1 (clone V18A), Coxsackievirus A1 (isolate KS-ZPH01F/XJ/CHN/2011), Coxsackievirus A11 (isolate CV-A11_66122), Coxsackievirus A13 (clone V4B), Coxsackievirus A13 (strain BAN01-10637), Coxsackievirus A19 (strain 2019103106/XX/CHN/2019), Coxsackievirus A19 (strain 8663), Coxsackievirus A20 (strain CAM1976), Coxsackievirus A21 (isolate 12MYKLU412), Coxsackievirus A21 (strain NIV17-608-2), Coxsackievirus A22 (strain 438913), Coxsackievirus A24 (strain 20693_84_CV-A24), Coxsackievirus A15 (strain G-9), Coxsackievirus A18 (strain CAM1972), Human rhinovirus A2 (strain 12L4), Human rhinovirus A2 (strain USA/2018/CA-RGDS-1062), Human rhinovirus A2 (X02316), Human rhinovirus A7 (strain ATCC VR-1117), Human rhinovirus A8 (strain ATCC VR-1118), Human rhinovirus A9 (isolate F01), Human rhinovirus A9 (isolate F02), Human rhinovirus A9 (strain ATCC VR-489), Human rhinovirus A10 (strain ATCC VR-1120), Human rhinovirus A11 (strain RvA11/USA/2021/XHZLKL), Human rhinovirus A11 (strain SCH-107), Human rhinovirus A11 (EF173414), Human rhinovirus A12 (isolate p211), Human rhinovirus A12 (EF173415), Human rhinovirus A13 (strain ATCC VR-1123), Human rhinovirus A13 (isolate F03), Human rhinovirus A15 (isolate 7002), Human rhinovirus A15 (DQ473493), Human rhinovirus A16 (isolate KC939), Human rhinovirus A16 (HRVPP), Human rhinovirus A18 (strain HRVA18/03/ZJ/CHN/2017), Human rhinovirus 18 (strain ATCC VR-1128), Human rhinovirus 19 (strain ATCC VR-1129), Human rhinovirus A20 (strain RvA20/USA/2021/B4Q4QT), Human rhinovirus A22 (strain RvA22/USA/2021/WBLGNP), Human Rhinovirus A23 (strain RvA23/USA/2021/JZHYZ6), Human rhinovirus A24 (strain RvA24/USA/2021/QZ8RX3), Human Rhinovirus A25 (strain RvA25/USA/2021/A8F6KW), Human Rhinovirus A28 (strain RvA28/USA/2021/ADMJHA), Human Rhinovirus A29 (strain RvA29/USA/2021/273658-4), Human rhinovirus A30 (strain MCL-18-H-1135), Human rhinovirus A31 (strain RvA31/USA/2021/273760-4), Human rhinovirus A32 (strain ATCC VR-1142), Human rhinovirus A33 (strain ATCC VR-330), Human rhinovirus A34 (strain ATCC VR-1144), Human rhinovirus A36 (DQ473505.1), Human rhinovirus A38 (strain ATCC VR-1148), Human rhinovirus A39 (strain ATCC VR-340), Human rhinovirus A40 (strain 7D5), Human rhinovirus A41 (strain SC9861), Human rhinovirus A43 (strain ATCC VR-1153), Human rhinovirus A44 (DQ473499), Human rhinovirus A45 (strain ATCC VR-1155), Human rhinovirus A46 (strain RvA46/USA/2021/6EEDHN), Human rhinovirus A47 (strain ATCC VR-1157), Human rhinovirus A49 (isolate F04), Human rhinovirus A50 (strain ATCC VR-517), Human rhinovirus A51 (strain ATCC VR-1161), Human rhinovirus A53 (DQ473507), Human rhinovirus A54 (strain ATCC VR-1164), Human rhinovirus A55 (DQ473511), Human rhinovirus A56 (strain ATCC VR-1166), Human rhinovirus A57 (isolate fs ship#1-hrv-57), Human rhinovirus A58 (strain ATCC VR-1168), Human rhinovirus A59 (strain 16-J2), Human rhinovirus A60 (strain ATCC VR-1473), Human rhinovirus A61 (strain SCH-99), Human rhinovirus A62 (strain ATCC VR-1172), Human rhinovirus A63 (strain ATCC VR-1173), Human rhinovirus A64 (strain ATCC VR-1174), Human rhinovirus A65 (strain ATCC VR-1175), Human rhinovirus A66 (strain ATCC VR-1176), Human rhinovirus A67 (strain ATCC VR-1177), Human rhinovirus A68 (strain ATCC VR-1178), Human rhinovirus A71 (strain ATCC VR-1181), Human rhinovirus A74 (DQ473494), Human rhinovirus A75 (DQ473510), Human rhinovirus A76 (strain ATCC VR-1186), Human rhinovirus A77 (strain ATCC VR-1187), Human Rhinovirus A78 (strain RvA78/USA/2021/177499), Human rhinovirus A80 (strain ATCC VR-1190), Human rhinovirus A81 (isolate F06), Human rhinovirus A82 (strain ATCC VR-1192), Human_rhinovirus A85 (strain RvA85/USA/2021/AR424A), Human rhinovirus A88 (DQ473504.1), Human rhinovirus A90 (strain ATCC VR-1291), Human rhinovirus A94 (strain ATCC VR-1295), Human rhinovirus A95 (strain ATCC VR-1301), Human rhinovirus A96 (strain ATCC VR-1296), Human rhinovirus A98 (strain RvA98/USA/2021/W58KP8), Human rhinovirus A100 (strain ATCC VR-1300), Human rhinovirus A101 (strain SC1124), Human rhinovirus A103 (strain MCL-18-H-1122), Human rhinovirus B3 (NC_038312.1), Human rhinovirus B4 (DQ473490.1), Human rhinovirus B5 (strain ATCC VR-485), Human rhinovirus B6 (DQ473486.1), Human rhinovirus B17 (EF173420), Human rhinovirus B26 (strain ATCC VR-1136), Human rhinovirus B35 (strain ATCC VR-1145), Human rhinovirus B37 (EF173423), Human rhinovirus B42 (strain ATCC VR-338), Human rhinovirus B48 (DQ473488), Human rhinovirus B52 (isolate F10), Human rhinovirus B69 (strain ATCC VR-1179), Human rhinovirus B70 (DQ473489), Human rhinovirus B72 (strain ATCC VR-1182), Human rhinovirus B79 (isolate ZB/CHN/18), Human rhinovirus B83 (strain ATCC VR-1193), Human rhinovirus B84 (strain ATCC VR-1194), Human rhinovirus B86 (strain ATCC VR-1196), Human rhinovirus B91 (strain RvB91/USA/2021/95333), Human rhinovirus B92 (strain ATCC VR-1293), Human rhinovirus B93 (EF173425), Human rhinovirus B97 (strain ATCC VR-1297), Human rhinovirus B99 (strain ATCC VR-1299), Human rhinovirus C2 (isolate 470389), Human rhinovirus C6 (strain RvC6/USA/2021/LCP8K8), Human rhinovirus C8 (strain RvC8/USA/2021/7N6PM0), Human rhinovirus C9 (strain RvC9/USA/2021/96D92H), Human rhinovirus C10 (strain QCE), Human rhinovirus C11 (strain SC9849), Human rhinovirus C12 (strain RvC12/USA/2021/044858), Human rhinovirus C15 (strain RvC15/USA/2021/SUSM75), Human rhinovirus C17 (strain RvC17/USA/2021/T3RVH2), Human rhinovirus C23 (strain RvC23/USA/2021/ULVLFU), Human rhinovirus C30 (strain USA/2015/CA-RGDS-1045), Human rhinovirus C31 (strain RvC31/USA/2021/B8JUE1), Human rhinovirus C32 (strain USA/CA/RGDS-2016-1008), Human rhinovirus C34 (strain RvC34/USA/2021/BYRST7), Human rhinovirus C35 (strain RvC35/USA/2021/70881), Human rhinovirus C36 (strain RvC36/USA/2021/PEXCU4), Human rhinovirus C39 (strain RvC39/USA/2021/71206), Human rhinovirus C40 (strain RvC40/USA/2021/70389), Human rhinovirus C41 (strain USA/CA/2016-RGDS-1006), Human rhinovirus C42 (strain RvC42/USA/2021/278730), Human rhinovirus C43 (strain SC174), Human rhinovirus C47 (isolate CA-RGDS-1001), Human rhinovirus C50 (strain human/Australia/SG1/2008), Human rhinovirus C51 (isolate LZ508), Human rhinovirus C54 (isolate D3490), Human rhinovirus C56 (strain RvC56/USA/2021/466615), Enterovirus E (isolate HeN-A2), Enterovirus F (isolate HeN-B62), Enterovirus G (EV-G/Pig/JPN/Kana-Uchi13/2019/G1_PL-CP), Enterovirus I Dromedary camel enterovirus (strain 19CC), Bovine enterovirus GX20-1, Goat enterovirus (isolate NMG-F37), Aimelvirus 1 (strain gpai001), Ampivirus A1 (strain NEWT/2013/HUN), Equine rhinitis A virus (strain PERV-1), Foot-and-mouth disease virus - type A (isolate A/BR19-16_08dpi_CB-RF), Foot-and-mouth disease virus - type Asia 1 (isolate Mazbi/QOL-UVAS-Pak/2006), Foot-and-mouth disease virus - type C (isolate KEN/1/2004), Foot-and-mouth disease virus O (isolate o6pirbright iso58), Foot-and-mouth disease virus - type SAT 1 (isolate TAN/3/80), Duck hepatitis A virus 1 (strain R85952), Turkey avisivirus (isolate USA-IN1), Bopivirus sp (strain bovine/TV-9682/2019-HUN), Encephalomyocarditis virus (ZM12/14), Human TMEV-like cardiovirus (NC_010810), Saffold virus 3 (NGT07-987), Human cosavirus A (strain AM326BRA-AM/2017), Cosavirus F (strain NGR_2017_NHP_CV), Canine picodicistrovirus (strain 209), Equine rhinitis B virus 1, Simian hepatitis A virus, Hepatovirus D2 (isolate KS111230Crimig2011), Rodent hepatovirus (KEF121Sigmas2012), Hepatovirus G2 (isolate FO1AF48Rhilan2010), Loch Leven virus (isolate MW12_1o), Hunnivirus 05VZ (isolate 05VZ-75-RAT099), Melegrivirus A (NC_023858), Canine picornavirus, Turdivirus 3, Pasivirus A3 (strain swine/Zsana1/2013/HUN), Passerivirus (sp. strain waxbill/DB01/HUN/2014), Wenling sharpspine skate picornavirus (strain DHBYCGS18742), Picornaviridae (sp. rodent/RI,/PicoV/FJ2015), Avian sapelovirus, Marmot sapelovirus 2 (strain HT6), Bat picornavirus (isolate BtPV/13585-58/M.dau/DK/2014), Bat picornavirus LMA6 (isolate DesRot/Peru/LMA6_F_DrPicoV), Sicinivirus A1 (isolate JSY), Sicinivirus A5 (strain RS/BR/2015/1), Sicinivirus (sp. isolate Environment/NLD/2019/VE_7_picoma_3), Porcine teschovirus 10 (strain Vir 460/88), Tremovirus A (isolate GDs29), Yili teratoscincus roborowskii picornavirus 1 (strain LPWC175499), Canine kobuvirus (US-PC0082), Feline kobuvirus (strain FK-13), Feline kobuvirus (strain WHJ-1), Kobuvirus (dog/AN211D/USA/2009), Murine kobuvirus 1 (isolate MKV1/NYC/2014/M014/0146), Kobuvirus sewage Kathmandu (isolate KoV-SewKTM), Bovine kobuvirus (strain IL35164), Kobuvirus cattle/Kagoshima-1-22-KoV/2014/JPN (Kagoshima-1-22-KoV/2014/JPN), Caprine kobuvirus (isolate MN1/2018), Ferret kobuvirus (isolate MpKoV38), Grey squirrel kobuvirus (isolate UK 2010), Marmot kobuvirus (strain HT9), Ovine kobuvirus (isolate SKoV-China/SWUN/AB 18/2019), Human parechovirus type 1 (PicoBank/HPeV1/a virus p123), Human parechovirus 3 (strain CAU14/2015/KR), Human parechovirus 4 (isolate K251176-02), Human parechovirus 5 (strain CT86-6760), Human parechovirus 5 (4112/SapporoC/July/2018), Human parechovirus 6 (strain: NII561-2000), Human parechovirus 6 (isolate AFW), Human parechovirus 7, Human parechovirus 14 (clone V3C), Human parechovirus 17 (isolate 157Chzj058), Human parechovirus 18 (isolate 11 Chzj 207), Human parechovirus 19 (isolate 67Chzj 11), Ljungan virus strain 145SL (isolate 145SLG), Ljungan virus M1146, Ljungan virus 64-7855, Rattus tanezumi parechovirus (strain Wencheng-Rt386-3), Parechovirus (sp. strain Parchzj-6), Baskerville virus, Bemisia tabaci picorna-like virus 1 (isolate CAU-Q1), British Admiral virus (isolate MW13_1o), Carfax virus, Chicken picornavirus 4 (isolate 5C), Chicken picornavirus 5 (isolate 27C), Chicken proventriculitis virus (isolate CPV/Korea/03), Zebrafish picornavirus-1 (strain NCSZCF/ZfPV/2015/North Carolina/USA), Duck picornavirus (duck/FC22/China/2017), Eotetranychus kankitus picorna-like virus (strain EKPLVabc9), Falcon picornavirus, Feline picornavirus (strain 661F), French Guiana picornavirus (isolate French_Guiana_Picornavirus), Leveillula taurica associated picorna-like virus 1 (isolate PM-A_)N31116), Moran virus, Mus musculus picornavirus (strain Wencheng-Mm283), Ovine picornavirus, Pigeon mesivirus 2 (strain pigeon/GALII5-PiMeV/2011/HUN), Red-necked stint Picornavirus B-like, Sphenigellan virus, Sphenimaju virus, Washington bat picornavirus, Waterwitch virus (isolate MW03_1o), Aphid lethal paralysis virus, Cricket paralysis virus, Drosophila C virus (strain EB), Homalodisca coagulata virus-1, Antheraea pernyi iflavirus (isolate LnApIV-02), Isla virus (strain Cx 1773-5), Chaetoceros socialis f. radians RNA virus, and Apple latent spherical virus.

The polynucleotides provided by the disclosure have the activity of initiating translation of the circular nucleic acid molecule, and can mediate an expression process of a protein in the circular nucleic acid molecule, which achieves highly efficient translation and expression of the protein and provides a good application basis for the application of the circular nucleic acid molecule.

In some embodiments, the disclosure provides a polynucleotide (i) having the activity of initiating translation of a circular nucleic acid molecule, where the polynucleotide includes a nucleotide sequence shown in any one of SEQ ID NOs: 1 to 548. Preferably, the polynucleotide includes a nucleotide sequence shown in SEQ ID NOs: 1, 2, 3, 4, 9, 10, 11, 13, 14, 15, 17, 18, 19, 20, 25, 26, 27, 28, 41, 42, 45, 46, 51, 56, 59, 72, 79, 91, 98, 101, 104, 106, 107, 110, 115, 116, 117, 118, 119, 122, 123, 125, 127, 129, 130, 135, 139, 165, 179, 180, 183, 186, 188, 198, 200, 215, 216, 217, 218, 219, 220, 221, 222, 223, 225, 227, 228, 229, 230, 231, 232, 233, 234, 235, 236, 239, 240, 242, 243, 244, 245, 246, 247, 248, 249, 250, 251, 252, 253, 254, 255, 256, 257, 258, 261, 262, 263, 264, 265, 266, 267, 268, 269, 270, 272, 273, 274, 275, 276, 278, 279, 280, 281, 282, 283, 284, 285, 286, 287, 289, 291, 293, 294, 296, 298, 301, 302, 303, 304, 305, 306, 307, 308, 309, 310, 311, 312, 314, 315, 317, 318, 319, 321, 322, 323, 324, 326, 329, 331, 332, 333, 334, 335, 336, 348, 385, 387, 389, 392, 393, 394, 395, 406, 436, 438, 439, 441, 445, 457, 460, 496, 504, 507, 509, 511, 514, and 534.

A polynucleotide shown in any sequence of SEQ ID NOs: 1 to 548 obtained via screening in the disclosure can recruit a ribosome in the circular nucleic acid molecule to initiate translation of the circular nucleic acid molecule. A polynucleotide shown in a preferred sequence mediates the protein expression level of the circular nucleic acid molecule to be significantly higher than that of CVB3 IRES, which can improve the expression level of the polypeptide and protein of interest, thereby providing abundant translation initiation elements for use of the circular nucleic acid molecule in preparing a protein, serving as vaccines, producing a therapeutic protein, serving as a means of gene therapy, etc.

Although the circular nucleic acid molecule has extremely high application potential in protein expression and prevention or treatment of clinical diseases, the sequences that can be used to initiate translation of circular nucleic acid molecules have not been found in large numbers. The screening method provided by the disclosure provides abundant translation initiation sequences for circular nucleic acid molecules, and has an important value for broadening industrial and clinical application of the circular nucleic acid molecule.

In some embodiments, the polynucleotide further includes a mutant sequence (ii) of any nucleotide sequence shown in (i), where the mutant sequence has a mutant nucleotide at one or more positions of any corresponding sequence shown in (i), and the mutant sequence has the activity of initiating translation of the circular nucleic acid molecule.

In the disclosure, the mutant sequence refers to a polynucleotide that contains a change (that is, substitution, insertion and/or deletion) at one or more (for example, several) positions relative to a "wild-type" or "comparative" nucleotide sequence, where the substitution means substituting a different nucleotide for a nucleotide occupying a position. Deletion refers to removal of a nucleotide occupying a certain position. Insertion refers to addition of a nucleotide at a position adjacent to and immediately following a nucleotide occupying a position.

In some specific embodiments, the mutant sequence includes one or more nucleotides deleted from or added to a 5' end of any corresponding nucleotide sequence shown in (i). In some specific embodiments, the mutant sequence includes one or more nucleotides deleted from or added to a 3' end of any corresponding nucleotide sequence shown in (i). In some specific embodiments, the mutant sequence includes one or more nucleotides deleted, added and/or substituted inside any corresponding nucleotide sequence shown in (i).

In the disclosure, the mutant sequence may have an increased activity of initiating translation of the circular nucleic acid molecule, or retained or at least partially retained activity of initiating translation of the circular nucleic acid molecule compared with a non-mutated nucleotide sequence. Specifically, as long as the mutated nucleotide does not cause loss of the mutant sequence's activity of initiating translation of the circular nucleic acid molecule, the mutant sequence falls within the scope of the disclosure.

In some embodiments, the polynucleotide having the activity of initiating translation of the circular nucleic acid molecule further includes: a nucleotide sequence that can be reversely complementary to a hybridized sequence of the nucleotide sequence shown in (i) or (ii) under a highly stringent hybridization condition or a very highly stringent hybridization condition and that has the activity of initiating translation of the circular nucleic acid molecule.

In some embodiments, the polynucleotide having the activity of initiating translation of the circular nucleic acid molecule further includes a nucleotide sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99% (including all ranges and percentages between these values) sequence identity with the nucleotide sequence shown in any one of (i) or (ii) and having the activity of initiating translation of the circular nucleic acid molecule.

In some embodiments, the disclosure provides use of the polynucleotide in at least one of (a₁)-(a₂):
(a₁) initiating translation of a circular nucleic acid molecule, or preparing a product for initiating translation of a circular nucleic acid molecule; and
(a₂) increasing a protein expression level of a circular nucleic acid molecule, or preparing a product for increasing a protein expression level of a circular nucleic acid molecule.

The polynucleotide provided by the disclosure is used for initiating protein translation of the circular nucleic acid molecule, and has high translation activity, thereby implementing stable and efficient expression of the protein of interest.

### Circular nucleic acid molecule

The circular nucleic acid molecule provided by the disclosure includes the polynucleotide shown in any sequence in (i). The circular nucleic acid molecule has high protein expression efficiency and have a great application potential in the fields such as industrial protein production, nucleic acid vaccines, expression of therapeutic proteins, and gene therapies.

In some embodiments, the circular nucleic acid molecule is a circular RNA molecule. More specifically, the circular nucleic acid molecule is a circular mRNA molecule including a coding region encoding a polypeptide of interest. The coding region of the circular mRNA molecule is operably linked to the polynucleotide having the activity of initiating translation of the circular nucleic acid molecule, thereby initiating the protein translation process of the circular mRNA molecule.

In some embodiments, the circular mRNA molecule further includes one or more of the following elements: a 5' spacer region, a 3' spacer region, a second exon, and a first exon.

In some preferred embodiments, the circular mRNA molecule includes the following sequentially linked elements: a second exon E2, a 5' spacer region, the polynucleotide having the activity of initiating translation of the circular nucleic acid molecule, a coding region, a 3' spacer region, and a first exon E1. In the disclosure, it is found that the circular mRNA molecule with this structure has an increased protein expression level after insertion of the polynucleotide provided by the disclosure.

In the disclosure, the coding region may contain a nucleotide sequence encoding any protein. The sequence of the coding region is not specifically limited in the present disclosure, which is set according to a type of to-be-expressed protein of interest.

In some specific embodiments, the 5' spacer region includes a nucleotide sequence shown in any one of SEQ ID NOs: 549-550, or a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in any one of SEQ ID NOs: 549-550.

In some specific embodiments, the 3' spacer region includes a nucleotide sequence shown in any one of SEQ ID NOs: 551-553, or a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in any one of SEQ ID NOs: 551-553.

In some specific embodiments, the first exon E1 includes a nucleotide sequence shown in SEQ ID NO: 554, or a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in SEQ ID NO: 554.

In some specific embodiments, the second exon E2 includes a nucleotide sequence shown in SEQ ID NO: 555, or a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in SEQ ID NO: 555.

The disclosure finds that nucleotide sequences of the foregoing elements can further promote a protein translation process of the circular mRNA molecule mediated by the polynucleotide, and improve the activity of initiating protein translation by the polynucleotide.

In some other embodiments, the circular nucleic acid molecule may also include other types of elements or element sequences, which is not specifically limited in the disclosure, as long as the polynucleotides shown in SEQ ID NOs: 1 to 548 in the disclosure can initiate protein translation of the circular nucleic acid molecule to achieve high-level expression of the protein.

In some embodiments, the disclosure provides a cyclization precursor nucleic acid molecule, which can be cyclized to form the circular nucleic acid molecule described above. Further, the cyclization precursor nucleic acid molecule is a cyclization precursor mRNA molecule.

In some specific embodiments, the cyclization precursor mRNA molecule further includes one or more of the following elements: a 5' homology arm, a 3' intron, a second exon, a 5' spacer region, a coding region, a 3' spacer region, a first exon, a 5' intron and a 3' homology arm.

In some specific embodiments, the cyclization precursor mRNA molecule includes the following sequentially linked elements:
a 5' homology arm, a 3' intron, a second exon, a 5' spacer region, the polynucleotide having the activity of initiating translation of the circular nucleic acid molecule, a coding region, a 3' spacer region, a first exon, a 5' intron and a 3' homology arm.

The cyclization precursor mRNA molecule is cyclized by the following process: via a ribozyme feature of the intron, under the trigger of GTP, a junction of the 5' intron and the first exon is broken; and a ribozyme cleavage of the first exon further attacks a junction of the 3' intron and the second exon, causing break of the junction, the 3' intron is dissociated, and the first exon and the second exon are connected to form the circular mRNA molecule.

In some specific embodiments, the 5' homology arm includes a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with a nucleotide sequence shown in any one of SEQ ID Nos: 558-559.

In some specific embodiments, the 3' homology arm includes a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with a nucleotide sequence shown in any one of SEQ ID Nos: 560-561.

In some specific embodiments, the 5' intron includes a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in any one of SEQ ID No: 556.

In some specific embodiments, the 3' intron includes a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% sequence identity with the nucleotide sequence shown in any one of SEQ ID No: 557.

In some embodiments, the disclosure provides a recombinant nucleic acid molecule capable of being transcribed to form the cyclization precursor mRNA molecule described above. To enable further transcription of the recombinant nucleic acid molecule to form the mRNA molecule, the recombinant nucleic acid molecule may also contain a regulatory sequence. For example, the regulatory sequence is a T7 promoter linked to the upstream of the 5' homology arm.

In some embodiments, the disclosure provides a recombinant expression vector including the recombinant nucleic acid molecule described above. Vectors connecting the recombinant nucleic acid molecules can be various types of vectors commonly used in the art, for example, a pUC57 plasmid, etc. Further, the recombinant nucleic acid molecule contains a restriction site, so that a linearized vector suitable for transcription is obtained after the recombinant expression vector is digested by the enzyme.

In some embodiments, the disclosure provides a recombinant host cell, including at least one of the circular mRNA molecule, the cyclization precursor mRNA molecule, the recombinant nucleic acid molecule, and the recombinant expression vector.

### Example

Other objectives, features and advantages of the disclosure will become obvious from the following detailed description. However, it should be understood that the detailed description and specific examples (while showing specific embodiments of the disclosure) are provided for explanatory purposes only. Because after reading the detailed descriptions, various changes and modifications made within the spirit and scope of the disclosure will become obvious to those skilled in the art.

The experimental techniques and methods used in this example are conventional technical methods unless otherwise specified. For example, the experimental methods in which specific conditions are not specified in the following examples are usually performed according to conventional conditions for example, conditions described in Sambrook et al., Molecular Cloning: A Laboratory Manual (New York: Cold Spring Harbor Laboratory Press, 1989), or conditions recommended by a manufacturer. The materials, reagents, and the like used in the examples are officially commercially available unless otherwise specified.

### Example 1: Screening of sequence having activity of initiating translation of circular nucleic acid molecule

(1) Nucleotide sequences derived from different species of viruses were obtained and used as a set of to-be-predicted sequences.
(2) A set of 583 sample IRES sequences of which the activity had been experimentally verified were downloaded from iresite database (http://www.iresite.org).
(3) One-hot encoding: to-be-encoded objects were determined as (1) a set of obtained to-be-predicted sequences, and (2) a set of selected IRES sequences, wherein the categorical variables were A, T, C, and G; and each sample had 4 features, and the features were converted into binary vectors for representation. Taking SEQ ID NO: 1 as an example, details are shown in Table 4 below:

**Table 4**

| | T | T | A | A | A | A | C | A | G | ... | C | A | C | A | T | C | A | A | A |
|---|---|---|---|---|---|---|---|---|---|---|---|---|---|---|---|---|---|---|---|
| A | 0 | 0 | 1 | 1 | 1 | 1 | 0 | 1 | 0 | ... | 0 | 1 | 0 | 1 | 0 | 0 | 1 | 1 | 1 |
| T | 1 | 1 | 0 | 0 | 0 | 0 | 0 | 0 | 0 | ... | 0 | 0 | 0 | 0 | 1 | 0 | 0 | 0 | 0 |
| C | 0 | 0 | 0 | 0 | 0 | 0 | 1 | 0 | 0 | ... | 1 | 0 | 1 | 0 | 0 | 1 | 0 | 0 | 0 |
| G | 0 | 0 | 0 | 0 | 0 | 0 | 0 | 0 | 1 | ... | 0 | 0 | 0 | 0 | 0 | 0 | 0 | 0 | 0 |

(4) Calculation of Levenshtein distances: Levenshtein distances between each to-be-predicted sequence and the selected 583 sample IRES sequences were calculated, and an average was taken. In calculative mathematics, the Levenshtein distance between two strings a and b satisfy that levab(i, j) = max(i, j), where if min(i, j) = 0, levab(i, j) = min(levab(i - 1, j) + 1, levab(i, j-1) + 1, and levab(i - 1, j-1) + 1) (ai != bj), where ai != bj is an indicator function. When ai != bj, a value is 1, otherwise, a value is 0. It should be noted that in the minimum item, a first part corresponds to a deletion operation (from a to b), a second part corresponds to an insertion operation, and a third part corresponds to a substitution operation. The average of the Levenshtein distances between the to-be-predicted sequences and the 583 sample IRES sequences was calculated. The maximum average was 1.0. If the average was greater than 0.5, it could be preliminarily determined that the to-be-predicted sequence could contain the IRES; if the average was greater than 0.75, it was determined that the to-be-predicted sequence highly likely contained the IRES. The average of the Levenshtein distances was shown in Table 5 below.

**Table 5**

| SEQ ID NO: | Species | Average of Levenshtein distances |
|---|---|---|
| 1 | Echovirus E1 (strain Farouk / ATCC VR-1038) | 0.5808049313271684 |
| 2 | Echovirus E2 (strain USA/2013-19511) | 0.6188037379332704 |
| 3 | Echovirus E3 (isolate JSev001) | 0.5000632986851516 |
| 4 | Echovirus E3 (strain 61246-70294) | 0.6082589761442534 |
| 5 | Echovirus E3 (strain 61247-622) | 0.6073517314258708 |
| 6 | Echovirus E3 (strain 61245-2710) | 0.6061754786067719 |
| 7 | Echovirus E3 (strain 63038-1131) | 0.6018930633212138 |
| 8 | Echovirus E3 (strain 63040-70881) | 0.5970295357872576 |
| 9 | Echovirus E3 (isolate HNWY-01) | 0.5136681381373834 |
| 10 | Echovirus E3 (isolate ECHO3_INMI1) | 0.48382071550949773 |
| 11 | Echovirus E3 (isolate Env_2016_Sep_E-3) | 0.5793434993451302 |
| 12 | Echovirus E3 (strain Sakhalin-11.293) | 0.5541478951256454 |
| 13 | Echovirus E3 (strain HAI/2016-23067A) | 0.5473101688541446 |
| 14 | Echovirus E3 (strain HAI/2016-23066) | 0.5527812726135902 |
| 15 | Echovirus E3 (strain HAI/2016-23065A) | 0.5667800957051863 |
| 16 | Echovirus E3 (strain HAI/2016-23061) | 0.565103313316246 |
| 17 | Echovirus E3 (strain HAI/2016-23056) | 0.5511865958122903 |
| 18 | Echovirus E3 (strain HAI/2016-23051A) | 0.5332834592896887 |
| 19 | Echovirus E3 (strain HAI/2016-23050) | 0.5433437375965232 |
| 20 | Echovirus E3 (isolate 123-R2) | 0.5412315202753394 |
| 21 | Echovirus E3 (strain Sakhalin/10_DU145) | 0.5748063226382968 |
| 22 | Echovirus E3 (strain Sakhalin/10_RD) | 0.5764759708465969 |
| 23 | Echovirus E3 (isolate E3/TOBR/018) | 0.6523338974338045 |
| 24 | Echovirus E4 (strain 2F5) | 0.5643061256681934 |
| 25 | Echovirus 4 (strain AUS250G) | 0.5652543471609274 |
| 26 | Echovirus E4 (strain Pesacek) | 0.5175196720569315 |
| 27 | Echovirus E5 | 0.6039594525829762 |
| 28 | Echovirus E6 | 0.6040261442378229 |
| 29 | Echovirus 9 (strain Barty) | 0.6225482743952616 |
| 30 | Echovirus 9 (strain Hill) | 0.48864035578803333 |
| 31 | Echovirus E11 | 0.49839484274883805 |
| 32 | Echovirus E12 | 0.6661344256078723 |
| 33 | Echovirus E13 (strain HAI/2017-23078B) | 0.5116509698669113 |
| 34 | Echovirus E13 (strain HAI/2016-23072) | 0.5322682925773098 |
| 35 | Echovirus E13 (strain HAI/2016-23073) | 0.5518852133130182 |
| 36 | Echovirus E13 (strain HAI/2016-23075) | 0.5711015376985186 |
| 37 | Echovirus E13 (strain HAI/2017-23082B) | 0.5047549476513821 |
| 38 | Echovirus E14 (strain RO-81-1-79) | 0.5517610733049713 |
| 39 | Echovirus E14 (isolate ETH_P19/E14_2016) | 0.5416219091902743 |
| 40 | Echovirus E14 (isolate NSW-V04-2012-ECHO14) | 0.7877088231180686 |
| 41 | Echovirus E14 (isolate E14/P843/2013/China) | 0.6311207338131573 |
| 42 | Echovirus E14 (isolate E14/P968/2013/China) | 0.619622313996729 |
| 43 | Echovirus E15 (strain CH 96-51) | 0.5875706239418529 |
| 44 | Echovirus E16 (isolate ETH_P4/E16_2016) | 0.5084421973726146 |
| 45 | Echovirus E16 (isolate E16/P85/2013/China) | 0.6072950786401917 |
| 46 | Echovirus E16 (strain Harrington) | 0.5539581839578673 |
| 47 | Echovirus 17 (strain CHHE-29) | 0.4830894420137125 |
| 48 | Echovirus E18 (isolate PC06/JS/CHN/2019) | 0.5674112910391006 |
| 49 | Echovirus E18 (strain E18/JXY2-2/2019) | 0.5913386342445188 |
| 50 | Echovirus E18 (isolate QD9/SD/CHN/2019) | 0.5967486267240393 |
| 51 | Echovirus E18 (isolate LJ/0530/2019) | 0.5669165361014139 |
| 52 | Echovirus E18 (strain 12J3) | 0.5323674807300197 |
| 53 | Echovirus E18 (strain USA/2015/CA-RGDS-1049) | 0.5718321627431914 |
| 54 | Echovirus E18 (isolate E18-221/HeB/CHN/2015) | 0.5749871390587905 |
| 55 | Echovirus E18 (strain 12G5) | 0.518938908507651 |
| 56 | Echovirus E18 (isolate E18-393/HeB/CHN/2015) | 0.5966532826722779 |
| 57 | Echovirus E18 (isolate E18-398/HeB/CHN/2015) | 0.5802033135408055 |
| 58 | Echovirus E18 (isolate E18-HeB 15-54462/HeB/CHN/2015) | 0.5943115754334534 |
| 59 | Echovirus E18 (isolate E18-HeB 15-54498/HeB/CHN/2015) | 0.6114826956352949 |
| 60 | Echovirus E18 (isolate ETH_P12/E18_2016) | 0.5599577313314069 |
| 61 | Echovirus E18 (isolate NSW-V13A-2008-ECHO18) | 0.8016918133770672 |
| 62 | Echovirus E18 (strain A83/YN/CHN/2016) | 0.6162734978883699 |
| 63 | Echovirus E18 (strain A86/YN/CHN/2016) | 0.5666784066223288 |
| 64 | Echovirus E18 (isolate Jena/ST9524/10) | 0.5893255734301206 |
| 65 | Echovirus E18 (isolate Jena/VI10227/10) | 0.6001690065872023 |
| 66 | Echovirus E18 (isolate Kor05-ECV18-054cn) | 0.6109617945798228 |
| 67 | Echovirus E19 (strain HAI/2016-23039B) | 0.5619266173651392 |
| 68 | Echovirus E19 (strain HAI/2016-23036D) | 0.5852261104020761 |
| 69 | Echovirus E19 (strain HAI/2016-23037D) | 0.5360399210418508 |
| 70 | Echovirus E19 (strain HAI/2016-23037E) | 0.5367222933761491 |
| 71 | Echovirus E19 (strain HAI/2016-23042B) | 0.5547631164415266 |
| 72 | Echovirus E19 (strain HAI/2016-23046B) | 0.5919939389506693 |
| 73 | Echovirus E19 (strain HAI/2016-23047) | 0.5975375363696883 |
| 74 | Echovirus E19 (strain HAI/2016-23054) | 0.5619266173651392 |
| 75 | Echovirus E19 (strain HAI/2016-23052) | 0.5651548841304406 |
| 76 | Echovirus E19 (strain HAI/2016-23053) | 0.5568186393967952 |
| 77 | Echovirus E19 (strain HAI/2016-23062D) | 0.5442751663714708 |
| 78 | Echovirus E19 (strain HAI/2016-23063B) | 0.5339339475591622 |
| 79 | Echovirus E19 (strain HAI/2016-23064B) | 0.5334519938961495 |
| 80 | Echovirus E19 (strain HAI/2016-23067B) | 0.5422485564948548 |
| 81 | Echovirus E19 (strain HAI/2016-23070B) | 0.5873800159040743 |
| 82 | Echovirus E19 (strain HAI/2017-23079) | 0.5896767177946751 |
| 83 | Echovirus E19 (strain HAI/2017-23081A) | 0.5525749211468359 |
| 84 | Echovirus E19 (isolate ETH_P3/E19_2016) | 0.6556927383023295 |
| 85 | Echovirus E19 (strain NGR_2014) | 0.6312425608990878 |
| 86 | Echovirus E19 (isolate PDV_BLR_IN) | 0.5143236489882879 |
| 87 | Echovirus E19 (strain Burke) | 0.6212483255693274 |
| 88 | Echovirus E19 (strain K/542/81) | 0.5779384310070684 |
| 89 | Echovirus E20 (isolate E20/TO/BR/016) | 0.549495873428977 |
| 90 | Echovirus E20 (strain HAE2016-23 03 8B) | 0.5375351921169472 |
| 91 | Echovirus E20 (strain HAI/2016-23041B) | 0.513256714606494 |
| 92 | Echovirus E20 (strain HAI/2016-23085B) | 0.5399463374966579 |
| 93 | Echovirus E20 (strain HAI/2016-23065C) | 0.5589240448799935 |
| 94 | Echovirus E20 (strain HAI/2016-23068B) | 0.5374206583984363 |
| 95 | Echovirus E20 (strain HAI/2016-23069) | 0.5215856312718054 |
| 96 | Echovirus E20 (strain HAI/2017-23080B) | 0.528269598790309 |
| 97 | Echovirus E20 (strain HAI/2017-23081B) | 0.5430769693666437 |
| 98 | Echovirus E20 (HAI/2016-23077B) | 0.565615067758941 |
| 99 | Echovirus E20 (strain HAI/2017-23083C) | 0.5432259671714722 |
| 100 | Echovirus E20 (strain KM-EV20-2010) | 0.6445794685904701 |
| 101 | Echovirus E20 (strain JV-1) | 0.5125551016507701 |
| 102 | Echovirus E21 (strain 553/YN/CHN/2013) | 0.5635612795804391 |
| 103 | Echovirus E21 (strain Farina) | 0.5158668453401536 |
| 104 | Echovirus E24 (strain VEN/2018-23086) | 0.615957202123764 |
| 105 | Echovirus E24 (isolate PZ18G/JS/20120703) | 0.6621440382199824 |
| 106 | Echovirus E24 (strain DeCamp) | 0.5934294468111005 |
| 107 | Echovirus E25 (strain USA/2016-19521) | 0.6822112112544876 |
| 108 | Echovirus E25 (strain USA/2018-23126) | 0.5597967905509564 |
| 109 | Echovirus E25 (strain 10-4339-2) | 0.600702055000706 |
| 110 | Echovirus E25 (strain USA/CA/RGDS-2017-1010) | 0.5162776722043619 |
| 111 | Echovirus E25 (isolate NSW-V07-2007-ECHO25) | 0.6023913581937407 |
| 112 | Echovirus E25 (isolate NSW-V08-2008-ECHO25) | 0.6336353171076778 |
| 113 | Echovirus E25 (isolate NSW-V09-2008-ECHO25) | 0.883906966620007 |
| 114 | Echovirus E25 (isolate NSW-V58-2010-ECHO25) | 0.8780882139795565 |
| 115 | Echovirus E25 (strain 61241-70868) | 0.564412311786525 |
| 116 | Echovirus E25 (strain E25/ZE-wly/Zhej iang/CHN/2005) | 0.6391212557009869 |
| 117 | Echovirus E25 (isolate Jena/AN1380/10) | 0.6101193067296762 |
| 118 | Echovirus E25 (strain XM0297) | 0.6288150695867872 |
| 119 | Echovirus E25 (strain E25/2010/CHN/BJ) | 0.6331686090146701 |
| 120 | Echovirus E25 (isolate E25SD2010CHN) | 0.7132777071268944 |
| 121 | Echovirus E25 (strain HN-2) | 0.6002392009789782 |
| 122 | Echovirus E25 (strain JV-4) | 0.5608386821308077 |
| 123 | Echovirus E26 (strain Coronel) | 0.6062654480897788 |
| 124 | Echovirus E27 (isolate ETH_P8/E27_2016) | 0.5156137700552272 |
| 125 | Echovirus E27 (strain Bacon) | 0.5324156384056804 |
| 126 | Echovirus E29 (strain HAI/2016-23048B) | 0.5106046557252641 |
| 127 | Echovirus E29 (strain JV-10) | 0.5676063967690148 |
| 128 | Echovirus E30 (isolate E30/TO/BR/032) | 0.5191346267944849 |
| 129 | Echovirus E30 (isolate TL12C/NM/CHN/2016) | 0.5408130119094549 |
| 130 | Echovirus E30 (isolate TL7C/NM/CHN/2016) | 0.5420959375494635 |
| 131 | Echovirus E30 (strain USA/2018-23125) | 0.536644633332944 |
| 132 | Echovirus E30 (Echo3 0/Hokkaido. JPN/21208/2017) | 0.4751706742638117 |
| 133 | Echovirus E30 (strain USA/2015/CA-RGDS-1046) | 0.6359793363771304 |
| 134 | Echovirus E30 (strain USA/2017/CA-RGDS-1048) | 0.48976987236468716 |
| 135 | Echovirus E30 (isolate B001/USA/2016) | 0.5503500355147808 |
| 136 | Echovirus E30 (strain 16-I10) | 0.5185927407158059 |
| 137 | Echovirus E30 (strain 1-B4-TW) | 0.6228628861449574 |
| 138 | Echovirus E30 (strain 2002-59) | 0.5932845071630329 |
| 139 | Echovirus E30 (strain KM/A363/09) | 0.581569350680876 |
| 140 | Echovirus E30 (isolate 1-MRS2013) | 0.47383274194638425 |
| 141 | Echovirus E30 (isolate 3-MRS2013) | 0.4913222932049281 |
| 142 | Echovirus E30 (isolate 4-MRS2013) | 0.5227575120062752 |
| 143 | Echovirus E30 (isolate 2012EM161) | 0.6416981198957746 |
| 144 | Echovirus E30 (isolate E30SD2010CHN) | 0.5874930044754398 |
| 145 | Echovirus E30 (isolate ECV30/GX10/05) | 0.6171243419257207 |
| 146 | Echovirus E30 (strain Kor08-ECV30) | 0.5901817224847268 |
| 147 | Echovirus E30 (isolate FDJS03_84) | 0.6117929305771026 |
| 148 | Echovirus 30 (strain Bastianni) | 0.6304113799969484 |
| 149 | Echovirus 31 (strain Caldwell) | 0.5835167998403462 |
| 150 | Echovirus 32 (strain PR-10) | 0.5381486644772421 |
| 151 | Echovirus E33 (strain YNK35/CHN/2013) | 0.5540823631079579 |
| 152 | Echovirus E33 (strain YNA12/CHN/2013) | 0.5546686912617399 |
| 153 | Human poliovirus 1 (isolate CHN-Hainan/93-2) | 0.46093472546403114 |
| 154 | Human poliovirus 1 (isolate RUS39223) | 0.4944504596055311 |
| 155 | Human poliovirus 1 (isolate Pak-1) | 0.4529764960438368 |
| 156 | Human poliovirus 1 (isolate TJK35363 clone 6) | 0.47550274864547154 |
| 157 | Human poliovirus 1 (strain 3788ALB96) | 0.49583982996764026 |
| 158 | Human poliovirus 1 (isolate CHN15115/Xinjiang/CHN/2011) | 0.47147797909732997 |
| 159 | Human poliovirus 1 (isolate 29690_c1) | 0.4863153346047116 |
| 160 | Human poliovirus 1 (strain NIE1018316) | 0.4888103555140552 |
| 161 | Human poliovirus 1 (isolate EGY1218587) | 0.505474818199679 |
| 162 | Human poliovirus 1 (isolate 558/BRA-PE/88) | 0.4403001742175432 |
| 163 | Human poliovirus 2 (isolate Env2008_E2450) | 0.38043403445965707 |
| 164 | Human poliovirus 2 (strain CHA1218985) | 0.504944926831137 |
| 165 | Human poliovirus 2 (isolate Env2008_E3218) | 0.4173046683916367 |
| 166 | Human poliovirus 2 (strain MAD-2593-11) | 0.52746373854172 |
| 167 | Human poliovirus 3 (strain PAK1019536) | 0.5010478884678368 |
| 168 | Human poliovirus 3 (isolate Env08_E2886) | 0.5149400086491789 |
| 169 | Human poliovirus 3 (strain SWI10947) | 0.5393583610003766 |
| 170 | Human poliovirus 3 (strain FIN84-2493) | 0.4766221231527159 |
| 171 | Human poliovirus 3 (strain USOL-D-bac) | 0.3807851977468085 |
| 172 | Enterovirus A71 (isolate 2019-EV- A71-R398) | 0.45928824230619214 |
| 173 | Enterovirus A71 (strain USA/2018-23296) | 0.4946164989680169 |
| 174 | Enterovirus A71 (strain 16L) | 0.48767133883437264 |
| 175 | Enterovirus A76 (strain 10-3291-2) | 0.5599856118331821 |
| 176 | Human enterovirus A76 (AY697458) | 0.5721179844840873 |
| 177 | Enterovirus A89 (strain KSYPH-TRMH22F/XJ/CHN/2011) | 0.6243150331320565 |
| 178 | Human enterovirus A89 (AY697459.1) | 0.6370139483603551 |
| 179 | Enterovirus A90 (strain 10-2879-1) | 0.6004341224919545 |
| 180 | Enterovirus A90 (isolate SCH05F/XJ/CHN/2011) | 0.5975333034151918 |
| 181 | Human enterovirus A90 (isolate 01336/SD/CHN/EV90) | 0.6043038181896778 |
| 182 | Human enterovirus A90 (AB 192877.1) | 0.6116112430729701 |
| 183 | Human enterovirus A90 (isolate F950027) | 0.643517724294421 |
| 184 | Human enterovirus 91 (AY697461.1) | 0.6048459802558553 |
| 185 | Human enterovirus A92 (strain RJG7) | 0.5853760319381408 |
| 186 | Simian enterovirus SV19 (strain NOLA-2) | 0.5544977376443397 |
| 187 | Simian enterovirus SV19 (isolate cg4006) | 0.568907052748546 |
| 188 | Simian enterovirus SV19 (strain M19s (P2)) | 0.6242828045157908 |
| 189 | Simian enterovirus SV43 (strain OM112t (P12)) | 0.4845942720425571 |
| 190 | Simian enterovirus SV46 (isolate cg5400) | 0.6454386639433694 |
| 191 | Simian enterovirus SV46 (strain RNM5) | 0.5922665552823908 |
| 192 | Enterovirus B69 (strain Toluca-1) | 0.5447702203495234 |
| 193 | Enterovirus B69 (isolate 15_491) | 0.5334464307221062 |
| 194 | Enterovirus B73 (isolate 088/SD/CHN/04) | 0.5271925358182022 |
| 195 | Human enterovirus B73 (isolate 2776-82) | 0.45862999756243844 |
| 196 | Human enterovirus 74 (strain Rikaze- 13 6/XZ/CHN/2010) | 0.47943329626637027 |
| 197 | Enterovirus B75 (isolate Y16/XZ/CHN/2007) | 0.529659619602786 |
| 198 | Enterovirus B75 (isolate 102/SD/CHN/97) | 0.523149183564562 |
| 199 | Enterovirus B75 (strain USA/OK85-10362) | 0.5872937895620794 |
| 200 | Human enterovirus B77 (strain USA/TX97-10394) | 0.5579681499833907 |
| 201 | Human enterovirus B77 (strain CF496-99) | 0.6247112360229483 |
| 202 | Human enterovirus B79 (strain 17-2255-1_E79) | 0.4979564834992029 |
| 203 | Human enterovirus B79 (AB426610.1) | 0.4979564834992029 |
| 204 | Human enterovirus B79 (strain USA/CA79-10384) | 0.5734561092760242 |
| 205 | Enterovirus B80 (isolate HT-LYKH203F/XJ/CHN/2011) | 0.5502864862184469 |
| 206 | Human enterovirus B80 (isolate HZ01/SD/CHN/2004) | 0.6102199651974916 |
| 207 | Enterovirus B81 (isolate 99279/XZ/CHN/1999) | 0.6273765538555169 |
| 208 | Human enterovirus B81 (strain USA/CA68-10389) | 0.5795917247161194 |
| 209 | Human enterovirus B82 (strain USA/CA64-10390) | 0.628152354260522 |
| 210 | Human enterovirus B83 (strain USA/CA76-10392) | 0.6830088828075495 |
| 211 | Enterovirus B83 (isolate 99245/XZ/CHN/1999) | 0.5031269090299197 |
| 212 | Enterovirus B83 (isolate AFP341-GD-CHN-2001) | 0.5236572112470147 |
| 213 | Enterovirus B83 (isolate 246/YN/CHN/08) | 0.6595326398455966 |
| 214 | Enterovirus B84 (strain GHA:BAR:TES/2017) | 0.4854150433063059 |
| 215 | Enterovirus B84 (isolate AFP452/GD/CHN/2004) | 0.492275836192338 |
| 216 | Human enterovirus B84 (isolate CIV2003-10603) | 0.5502736397479051 |
| 217 | Human enterovirus B85 (strain HTPS-MKLH04F/XJ/CHN/2011) | 0.5453661557001908 |
| 218 | Human enterovirus B85 (strain BAN00-10353) | 0.5692568631304266 |
| 219 | Human enterovirus B86 (strain BAN00-10354) | 0.45406533968630014 |
| 220 | Enterovirus B87 (isolate LY02/SD/CHN/2000) | 0.5859291472196817 |
| 221 | Enterovirus B88 (strain 11-4644-1) | 0.6059751516648656 |
| 222 | Human enterovirus B88 (strain BAN01-10398) | 0.5876178405925064 |
| 223 | Enterovirus B93 (isolate 99052/XZ/CHN/1999) | 0.5958473867612367 |
| 224 | Enterovirus B93 (isolate 38-03) | 0.6611988574125724 |
| 225 | Human enterovirus B97 (strain 99188/SD/CHN/1999/EV97) | 0.6090638980650727 |
| 226 | Human enterovirus B97 (strain DT94-0227) | 0.5855907778137233 |
| 227 | Human enterovirus B97 (strain BAN99-10355) | 0.5891395752114498 |
| 228 | Human enterovirus B98 (strain: T92-1499) | 0.5481295942421415 |
| 229 | Human enterovirus B100 (isolate BAN2000-10500) | 0.5615476816393387 |
| 230 | Human enterovirus B101 (strain CIV03-10361) | 0.5804558234312348 |
| 231 | Enterovirus B 106 (isolate AKS-AWT-AFP2F/XJ/CHN/2011) | 0.6111962521257411 |
| 232 | Human enterovirus 106 (isolate 148/YN/CHN/12) | 0.627848181236402 |
| 233 | Enterovirus C96 (strain VEN/2018-23123A) | 0.5239188987301402 |
| 234 | Enterovirus C96 (isolate 127/SD/CHN/1991) | 0.5431014836327113 |
| 235 | Enterovirus C96 (clone V13C) | 0.5335353378492713 |
| 236 | Enterovirus C99 (strain 10L1) | 0.44273607915910396 |
| 237 | Human enterovirus C104 (isolate kvv585-16-TS) | 0.534829532144603 |
| 238 | Human enterovirus C105 (strain USA/OK/2014-19362) | 0.5136168835701784 |
| 239 | Human enterovirus C116 (strain 126) | 0.5041249369599711 |
| 240 | Enterovirus C 117 (strain JX-C 117-40-2017) | 0.5089142278031911 |
| 241 | Human enterovirus C118 (isolate CQ5185) | 0.5327115465313895 |
| 242 | Human enterovirus D68 (strain Fermon) | 0.6406183150822587 |
| 243 | Enterovirus D68 (TBp-13-Ph209) | 0.6357935500071978 |
| 244 | Enterovirus D70 (strain JPN/1989-23292) | 0.48319438334610393 |
| 245 | Enterovirus D94 (strain ANG/2010-23293) | 0.6118996021578769 |
| 246 | Human enterovirus D94 (isolate 19/04) | 0.6563359275753122 |
| 247 | Enterovirus D111 (strain ANG/2010-23294) | 0.5699262010560427 |
| 248 | Enterovirus D111 (isolate D111-NGR-KAT-1263) | 0.6540324157649857 |
| 249 | Simian enterovirus J103 (isolate cg8227) | 0.5816105743551186 |
| 250 | Coxsackievirus A2 (isolate HN202009) | 0.5660415279272476 |
| 251 | Coxsackievirus A2 (isolate 16027) | 0.5570056987639195 |
| 252 | Coxsackievirus A2 (isolate CVA2-13 88-M14/XY/CHN/2017) | 0.588488871495302 |
| 253 | Coxsackievirus A2 (isolate CVA2/Shenzhen50/CHN/2012) | 0.5730736914008895 |
| 254 | Coxsackievirus A2 (strain 2260165) | 0.5673882504795857 |
| 255 | Coxsackievirus A4 (strain CA4/JX2204/2014) | 0.612479022791526 |
| 256 | Coxsackievirus A4 (isolate HK458564/2016) | 0.6593754344515906 |
| 257 | Coxsackievirus A5 (isolate CV-A5-3487-M14-XY-CHN-2017) | 0.5330698387701938 |
| 258 | Coxsackievirus A5 (strain CVA5/13164/HUN/2015) | 0.4796578730433841 |
| 259 | Coxsackievirus A6 (isolate DN1501) | 0.5804411533180829 |
| 260 | Coxsackievirus A6 (strain RYN-A1205) | 0.610277500494171 |
| 261 | Coxsackievirus A7 (strain MAD-3101-11) | 0.554535220828899 |
| 262 | Coxsackievirus A8 (isolate 13-467/GS/CHN/2013) | 0.6106897997489629 |
| 263 | Coxsackievirus A8 (isolate C177/CHW/AUS/2017) | 0.5801726038359443 |
| 264 | Coxsackievirus A8 (isolate CV-A8/P82/2013/China) | 0.586953851288419 |
| 265 | Human coxsackievirus A8 (strain Donovan) | 0.5150727919892554 |
| 266 | Coxsackievirus A10 (isolate TA111R) | 0.4524759463951004 |
| 267 | Coxsackievirus A10 (strain CA10/JX2545/2017) | 0.5428384858952928 |
| 268 | Coxsackievirus A12 (isolate D89) | 0.565045437938567 |
| 269 | Coxsackievirus A12 (strain QD-LXH53 5/SD/CHN/2009) | 0.5879470769607731 |
| 270 | Coxsackievirus A14 (strain MAD-72-07) | 0.532912909014806 |
| 271 | Coxsackievirus A14 (isolate SEN-14-254) | 0.48600953120323537 |
| 272 | Human coxsackievirus A14 (strain G-14) | 0.5715593648178132 |
| 273 | Coxsackievirus A16 (isolate AH17-18/AH/East/CHN/2017-02-12) | 0.572283259514582 |
| 274 | Coxsackievirus A16 (isolate CV-A16/HVN08.039_HA_GIANGVNM/2008) | 0.6277458261568424 |
| 275 | Coxsackievirus B1 (strain RO-98-1-74) | 0.5963608708457682 |
| 276 | Coxsackievirus B1 (strain CVB1/XM0108) | 0.6268768394234222 |
| 277 | Coxsackievirus B1 (strain B 1/Groningen/2011) | 0.6956909587709591 |
| 278 | Coxsackievirus B2 (strain 13-2380-2_B2) | 0.5121588584672281 |
| 279 | Coxsackievirus B2 (strain 14L) | 0.5566278173482062 |
| 280 | Coxsackievirus B2 (strain 08-749-Shimane08-JPN) | 0.6036711279221575 |
| 281 | Coxsackievirus B2 (strain RW41-2/YN/CHN/2012) | 0.5927153164349939 |
| 282 | Coxsackievirus B2 (isolate BCH314) | 0.6335429762723401 |
| 283 | Coxsackievirus B3 (isolate B307) | 0.609382492589016 |
| 284 | Coxsackievirus B3 (isolate 2001-5) | 0.6437150913791714 |
| 285 | Coxsackievirus B3 (isolate DH09Y/JS/2012) | 0.5841942032562798 |
| 286 | Coxsackievirus B4 (isolate B401) | 0.618892464759692 |
| 287 | Coxsackievirus B4 (isolate CV-B4/P11/2013/China) | 0.534810658553231 |
| 288 | Coxsackievirus B4 (isolate Edwards CB4) | 0.601591405889082 |
| 289 | Coxsackievirus B5 (isolate B501) | 0.5917236122059703 |
| 290 | Coxsackievirus B5 (strain USA/MI/2009-23030) | 0.588820040103409 |
| 291 | Coxsackievirus B6 (isolate 99148/XZ/CHN/1999) | 0.50141787779587 |
| 292 | Coxsackievirus B6 (strain LEV15) | 0.5095790788495197 |
| 293 | Coxsackievirus A9 (strain A744/YN/CHN/2009) | 0.5420268010852607 |
| 294 | Coxsackievirus A9 (isolate 2-MRS2013) | 0.6350156522901241 |
| 295 | Coxsackievirus A1 (clone V18A) | 0.5394405618905521 |
| 296 | Coxsackievirus A1 (isolate KS-ZPH01F/XJ/CHN/2011) | 0.51830044840028 |
| 297 | Coxsackievirus A11 (isolate CV-A11_66122) | 0.5310888269417202 |
| 298 | Coxsackievirus A13 (clone V4B) | 0.5490320929091147 |
| 299 | Coxsackievirus A13 (strain BAN01-10637) | 0.5669533986135938 |
| 300 | Coxsackievirus A19 (strain 2019103106/XX/CHN/2019) | 0.5700953710266742 |
| 301 | Coxsackievirus A19 (strain 8663) | 0.5401802576685366 |
| 302 | Coxsackievirus A20 (strain CAM1976) | 0.5065831156049192 |
| 303 | Coxsackievirus A21 (isolate 12MYKLU412) | 0.5016165072075285 |
| 304 | Coxsackievirus A21 (strain NIV17-608-2) | 0.5697204907511733 |
| 305 | Coxsackievirus A22 (strain 438913) | 0.4985049695836058 |
| 306 | Coxsackievirus A24 (strain 20693_84_CV-A24) | 0.5597840865484324 |
| 307 | Coxsackievirus A15 (strain G-9) | 0.4860516766145873 |
| 308 | Coxsackievirus A18 (strain CAM1972) | 0.5592051513670969 |
| 309 | Human rhinovirus A2 (strain 12L4) | 0.6086990950584722 |
| 310 | Human rhinovirus A2 (strain USA/2018/CA-RGDS-1062) | 0.5850583251521847 |
| 311 | Human rhinovirus A2 (X02316) | 0.6603437212679295 |
| 312 | Human rhinovirus A7 (strain ATCC VR-1117) | 0.6941714121155632 |
| 313 | Human rhinovirus A8 (strain ATCC VR-1118) | 0.6010836874691167 |
| 314 | Human rhinovirus A9 (isolate F01) | 0.6235082376098245 |
| 315 | Human rhinovirus A9 (isolate F02) | 0.65264278855691 |
| 316 | Human rhinovirus A9 (strain ATCC VR-489) | 0.645181918253583 |
| 317 | Human rhinovirus A10 (strain ATCC VR-1120) | 0.6409288123602587 |
| 318 | Human rhinovirus A11 (strain RvA1 1/USA/2021/XHZLKL) | 0.6338185597096168 |
| 319 | Human rhinovirus A11 (strain SCH-107) | 0.6403359605567032 |
| 320 | Human rhinovirus A11 (EF173414) | 0.6395014628823757 |
| 321 | Human rhinovirus A12 (isolate p211) | 0.6898313539110299 |
| 322 | Human rhinovirus A12 (EF173415) | 0.6712016699615532 |
| 323 | Human rhinovirus A13 (strain ATCC VR-1123) | 0.6763621443513593 |
| 324 | Human rhinovirus A13 (isolate F03) | 0.6662891838497392 |
| 325 | Human rhinovirus A15 (isolate 7002) | 0.6174221915751837 |
| 326 | Human rhinovirus A15 (DQ473493) | 0.7110001569419926 |
| 327 | Human rhinovirus A16 (isolate KC939) | 0.5581278567135982 |
| 328 | Human rhinovirus A16 (HRVPP) | 0.5789455711377887 |
| 329 | Human rhinovirus A18 (strain HRVA18/03/ZJ/CHN/2017) | 0.6719505462668024 |
| 330 | Human rhinovirus 18 (strain ATCC VR-1128) | 0.6698880033189915 |
| 331 | Human rhinovirus 19 (strain ATCC VR-1129) | 0.5687796185785023 |
| 332 | Human rhinovirus A20 (strain RvA20/USA/2021B4Q4QT) | 0.7373440855592669 |
| 333 | Human rhinovirus A22 (strain RvA22/USA/2021/WBLGNP) | 0.6340294722121228 |
| 334 | Human Rhinovirus A23 (strain RvA23/USA/2021/JZHYZ6) | 0.5980563343450229 |
| 335 | Human rhinovirus A24 (strain RvA24/USA/2021/QZ8RX3) | 0.7097046515083459 |
| 336 | Human Rhinovirus A25 (strain RvA25/USA/2021/A8F6KW) | 0.641808457483705 |
| 337 | Human Rhinovirus A28 (strain RvA28/USA/2021/ADMJHA) | 0.6671287008947643 |
| 338 | Human Rhinovirus A29 (strain RvA29/USA/2021/273 65 8-4) | 0.664814106173672 |
| 339 | Human rhinovirus A30 (strain MCL-18-H-1135) | 0.687113800664511 |
| 340 | Human rhinovirus A31 (strain RvA31/USA/2021/273760-4) | 0.673206538723218 |
| 341 | Human rhinovirus A32 (strain ATCC VR-1142) | 0.641296258404341 |
| 342 | Human rhinovirus A33 (strain ATCC VR-330) | 0.6099256264329906 |
| 343 | Human rhinovirus A34 (strain ATCC VR-1144) | 0.6636464775561838 |
| 344 | Human rhinovirus A36 (DQ473505.1) | 0.6606183633492794 |
| 345 | Human rhinovirus A38 (strain ATCC VR-1148) | 0.6780677904469626 |
| 346 | Human rhinovirus A39 (strain ATCC VR-340) | 0.5426717778888348 |
| 347 | Human rhinovirus A40 (strain 7D5) | 0.6924487889824577 |
| 348 | Human rhinovirus A41 (strain SC9861) | 0.7000947554928159 |
| 349 | Human rhinovirus A43 (strain ATCC VR-1153) | 0.6506184377433443 |
| 350 | Human rhinovirus A44 (DQ473499) | 0.7033357020444904 |
| 351 | Human rhinovirus A45 (strain ATCC VR-1155) | 0.5919359167635694 |
| 352 | Human rhinovirus A46 (strain RvA46/USA/2021/6EEDHN) | 0.707417026396848 |
| 353 | Human rhinovirus A47 (strain ATCC VR-1157) | 0.693303085280375 |
| 354 | Human rhinovirus A49 (isolate F04) | 0.6999255319324668 |
| 355 | Human rhinovirus A50 (strain ATCC VR-517) | 0.6209333930491198 |
| 356 | Human rhinovirus A51 (strain ATCC VR-1161) | 0.6112131964489288 |
| 357 | Human rhinovirus A53 (DQ473507) | 0.6405586364661005 |
| 358 | Human rhinovirus A54 (strain ATCC VR-1164) | 0.7369458660398449 |
| 359 | Human rhinovirus A55 (DQ473511) | 0.5996301494815367 |
| 360 | Human rhinovirus A56 (strain ATCC VR-1166) | 0.7068649165104073 |
| 361 | Human rhinovirus A57 (isolate fs ship#1-hrv-57) | 0.6939098322543827 |
| 362 | Human rhinovirus A58 (strain ATCC VR-1168) | 0.6619016528440018 |
| 363 | Human rhinovirus A59 (strain 16-J2) | 0.619082076496769 |
| 364 | Human rhinovirus A60 (strain ATCC VR-1473) | 0.6232091602878583 |
| 365 | Human rhinovirus A61 (strain SCH-99) | 0.6193983920541493 |
| 366 | Human rhinovirus A62 (strain ATCC VR-1172) | 0.6362515976952244 |
| 367 | Human rhinovirus A63 (strain ATCC VR-1173) | 0.586276987578181 |
| 368 | Human rhinovirus A64 (strain ATCC VR-1174) | 0.6500992322829021 |
| 369 | Human rhinovirus A65 (strain ATCC VR-1175) | 0.5957513866408007 |
| 370 | Human rhinovirus A66 (strain ATCC VR-1176) | 0.6151296723206161 |
| 371 | Human rhinovirus A67 (strain ATCC VR-1177) | 0.7145838589400889 |
| 372 | Human rhinovirus A68 (strain ATCC VR-1178) | 0.6636916580444769 |
| 373 | Human rhinovirus A71 (strain ATCC VR-1181) | 0.6467369610543777 |
| 374 | Human rhinovirus A74 (DQ473494) | 0.7089676684681712 |
| 375 | Human rhinovirus A75 (DQ473510) | 0.5682285342979287 |
| 376 | Human rhinovirus A76 (strain ATCC VR-1186) | 0.6490012912556992 |
| 377 | Human rhinovirus A77 (strain ATCC VR-1187) | 0.7207353185073148 |
| 378 | Human Rhinovirus A78 (strain RvA78/USA/2021/177499) | 0.6349810678058351 |
| 379 | Human rhinovirus A80 (strain ATCC VR-1190) | 0.7567640534727206 |
| 380 | Human rhinovirus A81 (isolate F06) | 0.5902285748036626 |
| 381 | Human rhinovirus A82 (strain ATCC VR-1192) | 0.6184752333617372 |
| 382 | Human_rhinovirus A85 (strain RvA85/USA/2021/AR424A) | 0.6911259381314915 |
| 383 | Human rhinovirus A88 (DQ473504.1) | 0.6290888593406224 |
| 384 | Human rhinovirus A90 (strain ATCC VR-1291) | 0.6792783261914022 |
| 385 | Human rhinovirus A94 (strain ATCC VR-1295) | 0.6712198375496936 |
| 386 | Human rhinovirus A95 (strain ATCC VR-1301) | 0.5711450262170426 |
| 387 | Human rhinovirus A96 (strain ATCC VR-1296) | 0.5649887624921948 |
| 388 | Human rhinovirus A98 (strain RvA98/USA/2021/W58KP8) | 0.651281570455754 |
| 389 | Human rhinovirus A100 (strain ATCC VR-1300) | 0.7402268410622288 |
| 390 | Human rhinovirus A101 (strain SC1124) | 0.6700188648996388 |
| 391 | Human rhinovirus A103 (strain MCL-18-H-1122) | 0.6285775904071377 |
| 392 | Human rhinovirus B3 (NC_038312.1) | 0.6957073463601183 |
| 393 | Human rhinovirus B4 (DQ473490.1) | 0.6523603148752493 |
| 394 | Human rhinovirus B5 (strain ATCC VR-485) | 0.6314849776516597 |
| 395 | Human rhinovirus B6 (DQ473486.1) | 0.7058295528619624 |
| 396 | Human rhinovirus B17 (EF 173420) | 0.6137949416494946 |
| 397 | Human rhinovirus B26 (strain ATCC VR-1136) | 0.6323383424251291 |
| 398 | Human rhinovirus B35 (strain ATCC VR-1145) | 0.6178350517817417 |
| 399 | Human rhinovirus B37 (EF173423) | 0.6504143837112901 |
| 400 | Human rhinovirus B42 (strain ATCC VR-338) | 0.6067030654533153 |
| 401 | Human rhinovirus B48 (DQ473488) | 0.5967825023086031 |
| 402 | Human rhinovirus B52 (isolate F10) | 0.5283441929152388 |
| 403 | Human rhinovirus B69 (strain ATCC VR-1179) | 0.5650162115124282 |
| 404 | Human rhinovirus B70 (DQ473489) | 0.5271324517314294 |
| 405 | Human rhinovirus B72 (strain ATCC VR-1182) | 0.6840645186069668 |
| 406 | Human rhinovirus B79 (isolate ZB/CHN/18) | 0.634167704109742 |
| 407 | Human rhinovirus B83 (strain ATCC VR-1193) | 0.6468347349735741 |
| 408 | Human rhinovirus B84 (strain ATCC VR-1194) | 0.6040703959556961 |
| 409 | Human rhinovirus B86 (strain ATCC VR-1196) | 0.6758180164057123 |
| 410 | Human rhinovirus B91 (strain RvB91/USA/2021/95333) | 0.5715717789485494 |
| 411 | Human rhinovirus B92 (strain ATCC VR-1293) | 0.5941218825178537 |
| 412 | Human rhinovirus B93 (EF173425) | 0.6862621572627255 |
| 413 | Human rhinovirus B97 (strain ATCC VR-1297) | 0.6830675238813152 |
| 414 | Human rhinovirus B99 (strain ATCC VR-1299) | 0.7423360352063163 |
| 415 | Human rhinovirus C2 (isolate 470389) | 0.534776396667412 |
| 416 | Human rhinovirus C6 (strain RvC6/USA/2021/LCP8K8) | 0.5807370971985787 |
| 417 | Human rhinovirus C8 (strain RvC8/USA/2021/7N6PM0) | 0.6248091989000637 |
| 418 | Human rhinovirus C9 (strain RvC9/USA/2021/96D92H) | 0.5990726492043625 |
| 419 | Human rhinovirus C10 (strain QCE) | 0.6518836182697529 |
| 420 | Human rhinovirus C11 (strain SC9849) | 0.543132357353825 |
| 421 | Human rhinovirus C12 (strain RvC 12/USA/2021/044858) | 0.608778813515426 |
| 422 | Human rhinovirus C15 (strain RvC 15/USA/2021/SUSM75) | 0.5438538174952772 |
| 423 | Human rhinovirus C17 (strain RvC 17/USA/2021/T3RVH2) | 0.5997166499256588 |
| 424 | Human rhinovirus C23 (strain RvC23/USA/2021/ULVLFU) | 0.5931273430822197 |
| 425 | Human rhinovirus C30 (strain USA/2015/CA-RGDS-1045) | 0.5587476022869116 |
| 426 | Human rhinovirus C31 (strain RvC31/USA/2021/B8JUE1) | 0.5419799360494493 |
| 427 | Human rhinovirus C32 (strain USA/CA/RGDS-2016-1008) | 0.5809228187499759 |
| 428 | Human rhinovirus C34 (strain RvC34/USA/2021BYRST7) | 0.7219555207590616 |
| 429 | Human rhinovirus C35 (strain RvC35/USA/2021/70881) | 0.6066565786094078 |
| 430 | Human rhinovirus C36 (strain RvC36/USA/2021/PEXCU4) | 0.4569698471657656 |
| 431 | Human rhinovirus C39 (strain RvC39/USA/2021/71206) | 0.4569698471657656 |
| 432 | Human rhinovirus C40 (strain RvC40/USA/2021/703 89) | 0.534776396667412 |
| 433 | Human rhinovirus C41 (strain USA/CA/2016-RGDS-1006) | 0.5739885946964087 |
| 434 | Human rhinovirus C42 (strain RvC42/USA/2021/278730) | 0.4569698471657656 |
| 435 | Human rhinovirus C43 (strain SC174) | 0.5200188711674328 |
| 436 | Human rhinovirus C47 (isolate CA-RGDS-1001) | 0.43573353438827417 |
| 437 | Human rhinovirus C50 (strain human/Australia/SG1/2008) | 0.42901107112939346 |
| 438 | Human rhinovirus C51 (isolate LZ508) | 0.476075418202231 |
| 439 | Human rhinovirus C54 (isolate D3490) | 0.5541056091187622 |
| 440 | Human rhinovirus C56 (strain RvC56/USA/2021/466615) | 0.5893480275172868 |
| 441 | Enterovirus E (isolate HeN-A2) | 0.6910243778798317 |
| 442 | Enterovirus F (isolate HeN-B62) | 0.6827104751262314 |
| 443 | Enterovirus G (EV-G/Pig/JPN/Kana-Uchi13/2019/G1_PL-CP) | 0.5333126783614752 |
| 444 | Enterovirus I Dromedary camel enterovirus (strain 19CC) | 0.6803640313322592 |
| 445 | Bovine enterovirus GX20-1 | 0.6999032547035025 |
| 446 | Goat enterovirus (isolate NMG-F37) | 0.5749860025515109 |
| 447 | Aimelvirus 1 (strain gpai001) | 0.6201715674199075 |
| 448 | Ampivirus A1 (strain NEWT/2013/HLTN) | 0.9323539719175006 |
| 449 | Equine rhinitis A virus (strain PERV-1) | 0.3831705530970938 |
| 450 | Foot-and-mouth disease virus - type A (isolate A/BR19-16_08dpi_CB-RF) | 0.3723932214177325 |
| 451 | Foot-and-mouth disease virus - type Asia 1 (isolate Mazbi/QOL-UVAS-Pak/2006) | 0.39597911530407054 |
| 452 | Foot-and-mouth disease virus - type C (isolate KEN/1/2004) | 0.4116994640832622 |
| 453 | Foot-and-mouth disease virus O (isolate o6pirbright iso58) | 0.37162203822167583 |
| 454 | Foot-and-mouth disease virus - type SAT 1 (isolate TAN/3/80) | 0.5254343782017207 |
| 455 | Duck hepatitis A virus 1 (strain R85952) | 0.6275181632524537 |
| 456 | Turkey avisivirus (isolate USA-IN1) | 0.6604368143907475 |
| 457 | Bopivirus sp (strain bovine/TV- 9682/2019-HUN) | 0.6136148346058375 |
| 458 | Encephalomyocarditis virus (ZM12/14) | 0.5759407101057598 |
| 459 | Human TMEV-like cardiovirus (NC_010810) | 0.6160440238325338 |
| 460 | Saffold virus 3 (NGT07-987) | 0.5785142657527343 |
| 461 | Human cosavirus A (strain AM326/BRA-AM/2017) | 0.6459214807126546 |
| 462 | Cosavirus F (strain NGR_2017_NHP_CV) | 0.681298284413891 |
| 463 | Canine picodicistrovirus (strain 209) | 0.7121602455273517 |
| 464 | Equine rhinitis B virus 1 | 0.6446522725894651 |
| 465 | Simian hepatitis A virus | 0.8882930616152281 |
| 466 | Hepatovirus D2 (isolate KS111230Crimig2011) | 0.8065465144168569 |
| 467 | Rodent hepatovirus (KEF121Sigmas2012) | 0.8621242698393188 |
| 468 | Hepatovirus G2 (isolate FO1AF48Rhilan2010) | 0.5072492850339075 |
| 469 | Loch Leven virus (isolate MW12_1o) | 0.4915700746191962 |
| 470 | Hunnivirus 05VZ (isolate 05VZ-75-RAT099) | 0.5798312138955524 |
| 471 | Melegrivirus A (NC _ 023858) | 0.5007866812621884 |
| 472 | Canine picornavirus | 0.585517073705111 |
| 473 | Turdivirus 3 | 0.5670044734269162 |
| 474 | Pasivirus A3 (strain swine/Zsana1/2013/HUN) | 0.554440780148236 |
| 475 | Passerivirus (sp. strain waxbill/DB01/HUN/2014) | 0.6756960353915241 |
| 476 | Wenling sharpspine skate picornavirus (strain DHBYCGS18742) | 0.8711180982997228 |
| 477 | Picornaviridae (sp. rodent/RL/PicoV/FJ2015) | 0.5044225012290093 |
| 478 | Avian sapelovirus | 0.5610691331462271 |
| 479 | Marmot sapelovirus 2 (strain HT6) | 0.42989625425608563 |
| 480 | Bat picornavirus (isolate BtPV/ 13 5 85-5 8/M. dau/DK/2014) | 0.7910329489378202 |
| 481 | Bat picornavirus LMA6 (isolate DesRot/Peru/LMA6_F_DrPicoV) | 0.41126703719410074 |
| 482 | Sicinivirus A1 (isolate JSY) | 0.6617934019225871 |
| 483 | Sicinivirus A5 (strain RS/BR/2015/1) | 0.8774637425411811 |
| 484 | Sicinivirus (sp. isolate Environment/NLD/2019/VE_7_picorna_3) | 0.7127568022773857 |
| 485 | Porcine teschovirus 10 (strain Vir 460/88) | 0.6603721488740731 |
| 486 | Tremovirus A (isolate GDs29) | 0.6426327538163137 |
| 487 | Yili teratoscincus roborowskii picornavirus 1 (strain LPWC 175499) | 0.6213002855664539 |
| 488 | Canine kobuvirus (US-PC0082) | 0.5323498073549009 |
| 489 | Feline kobuvirus (strain FK-13) | 0.5286234433047534 |
| 490 | Feline kobuvirus (strain WHJ-1) | 0.5257408247386066 |
| 491 | Kobuvirus (dog/AN211D/USA/2009) | 0.5766853662781989 |
| 492 | Murine kobuvirus 1 (isolate MKV 1/NYC/2014/M014/0146) | 0.4765019774903171 |
| 493 | Kobuvirus sewage Kathmandu (isolate KoV-SewKTM) | 0.03514619162735339 |
| 494 | Bovine kobuvirus (strain IL35164) | 0.5715857791556381 |
| 495 | Kobuvirus cattle/Kagoshima-1-22-KoV/2014/JPN (Kagoshima-1-22-KoV/2014/JPN) | 0.7456779628201752 |
| 496 | Caprine kobuvirus (isolate MN1/2018) | 0.7708151827420604 |
| 497 | Ferret kobuvirus (isolate MpKoV38) | 0.5161622299258443 |
| 498 | Grey squirrel kobuvirus (isolate UK 2010) | 0.6824243956373283 |
| 499 | Marmot kobuvirus (strain HT9) | 0.5330323362306334 |
| 500 | Ovine kobuvirus (isolate SKoV- China/SWUN/AB 18/2019) | 0.5821128962826022 |
| 501 | Human parechovirus type 1 (PicoBank/HPeV1/a virus p123) | 0.6436236371421008 |
| 502 | Human parechovirus 3 (strain CAU14/2015/KR) | 0.5849548700178346 |
| 503 | Human parechovirus 4 (isolate K251176-02) | 0.6405392188756479 |
| 504 | Human parechovirus 5 (strain CT86-6760) | 0.5232472533461368 |
| 505 | Human parechovirus 5 (4112/SapporoC/July/2018) | 0.5851346304628351 |
| 506 | Human parechovirus 6 (strain: NII561-2000) | 0.6015672857195756 |
| 507 | Human parechovirus 6 (isolate AFW) | 0.5357912855744474 |
| 508 | Human parechovirus 7 | 0.6181992709124706 |
| 509 | Human parechovirus 14 (clone V3C) | 0.625122665026285 |
| 510 | Human parechovirus 17 (isolate 157Chzj058) | 0.6671483525005787 |
| 511 | Human parechovirus 18 (isolate 11Chzj207) | 0.6291761917207371 |
| 512 | Human parechovirus 19 (isolate 67Chzj 11) | 0.8063714501003619 |
| 513 | Ljungan virus strain 145SL (isolate 145SLG) | 0.6987317991060082 |
| 514 | Ljungan virus M1146 | 0.6504659004799125 |
| 515 | Ljungan virus 64-7855 | 0.6223916484590848 |
| 516 | Rattus tanezumi parechovirus (strain Wencheng-Rt386-3) | 0.5596739988540328 |
| 517 | Parechovirus (sp. strain Parchzj-6) | 0.5484680905353069 |
| 518 | Baskerville virus | 0.5798218777631448 |
| 519 | Bemisia tabaci picorna-like virus 1 (isolate CAU-Q1) | 0.9186018006034752 |
| 520 | British Admiral virus (isolate MW13_1o) | 0.7526180196431712 |
| 521 | Carfax virus | 0.8170327013008536 |
| 522 | Chicken picornavirus 4 (isolate 5C) | 0.527590817500035 |
| 523 | Chicken picornavirus 5 (isolate 27C) | 0.5674808304619496 |
| 524 | Chicken proventriculitis virus (isolate CPV/Korea/03) | 0.45784182696650955 |
| 525 | Zebrafish picornavirus-1 (strain NCSZCF/ZfPV/2015/North Carolina/USA) | 0.6522458425852629 |
| 526 | Duck picornavirus (duck/FC22/China/2017) | 0.9186018006034752 |
| 527 | Eotetranychus kankitus picorna-like virus (strain EKPLVabc9) | 0.9196267660332578 |
| 528 | Falcon picornavirus | 0.6430851499966271 |
| 529 | Feline picornavirus (strain 661F) | 0.44267982288545704 |
| 530 | French Guiana picornavirus (isolate French_Guiana_Picornavirus) | 0.6619949125640623 |
| 531 | Leveillula taurica associated picorna-like virus 1 (isolate PM-A_DN31116) | 0.9022087883082625 |
| 532 | Moran virus | 0.6323709195044684 |
| 533 | Mus musculus picornavirus (strain Wencheng-Mm283) | 0.25196993122774 |
| 534 | Ovine picornavirus | 0.6705311251552103 |
| 535 | Pigeon mesivirus 2 (strain pigeon/GALII5-PiMeV/2011/HUN) | 0.5926908737190554 |
| 536 | Red-necked stint Picornavirus B-like | 0.7090833184293232 |
| 537 | Sphenigellan virus | 0.7200148179128709 |
| 538 | Sphenimaju virus | 0.4798727791622594 |
| 539 | Washington bat picornavirus | 0.5869710349285941 |
| 540 | Waterwitch virus (isolate MW03_1o) | 0.5262417865726503 |
| 541 | Aphid lethal paralysis virus | 0.894268683930682 |
| 542 | Cricket paralysis virus | 0.6279496160894118 |
| 543 | Drosophila C virus (strain EB) | 0.8504610251517164 |
| 544 | Homalodisca coagulata virus-1 | 0.45695353371742126 |
| 545 | Antheraea pernyi iflavirus (isolate LnApIV-02) | 0.9233007083916378 |
| 546 | Isla virus (strain Cx 1773-5) | 0.9177885606469574 |
| 547 | Chaetoceros socialis f. radians RNA virus | 0.8429611238455599 |
| 548 | Apple latent spherical virus | 0.8733428004594727 |

### Example 2: Verification of IRES activity of to-be-predicted sequences

### 2.1 Plasmid construction

Plasmids containing different IRES elements and coding genes eGFP were constructed, and this step was entrusted to Nanjing Genscript Biotech Corporation for gene synthesis and cloning. A DNA vector of constructed circular RNA included a T7 promoter, a 5' homology arm (SEQ ID NO: 558), a 3' intron (SEQ ID NO: 557), a second exon E2 (SEQ ID NO: 555), a 5' spacer region (SEQ ID NO: 549), an IRES element, an eGFP protein coding region sequence, a 3' spacer region (SEQ ID NO: 551), a first exon E1 (SEQ ID NO: 554), a 5' intron (SEQ ID NO: 556), a 3' homology arm (SEQ ID NO: 560), and a restriction site XbaI that can be used for plasmid linearization. The obtained gene fragment was connected to a pUC57 vector.

### 2.2 Preparation of linear plasmid template

### 2.2.1 Plasmid extraction

(1) Stab culture bacteria synthesized in vitro were activated under 37°C at 220 rpm for 3 to 4 hours.
(2) An activated bacterial solution was taken for amplification culture under a culture condition of 37°C at 220 rpm overnight.
(3) A plasmid was extracted (a Tiangen endotoxin-free small amount Midiprep Kit), and an OD value was measured.

### 2.2.2 Plasmid digestion

The plasmid prepared in the foregoing step 2.2.1 was digested with a XbaI single digestion.

Enzyme digestion system:

**Table 6**

| Reagent | Volume |
|---|---|
| Plasmid | 10 µg |
| XbaI restriction endonuclease | 5 µL |
| 10 × cutsmart buffer | 30µL |
| Nuclease free water | Total 300 µL |

Enzyme digestion was conducted at 37°C overnight. A universal DNA gel extraction kit (Tiangen Biotech (Beijing) Co., Ltd.) was used to recover an enzyme-digested product, the OD value was measured, and the enzyme-digested product was identified via 1% agarose gel electrophoresis. A purified linear plasmid template was used for in vitro transcription.

### 2.2.3 Preparation of mRNA via in vitro transcription

### 2.2.3.1 Preparation of circular mRNA via one-step transcription and cyclization

1) An in vitro transcription reaction was conducted, and the system was as follows:

**Table 7**

| Reagent | Volume |
|---|---|
| 10 × Reaction buffer | 2 µL |
| ATP (20 mM) | 2 µL |
| CTP (20mM) | 2 µL |
| UTP (20mM) | 2 µL |
| GTP (20mM) | 2 µL |
| Linearized DNA template | 600 ng |
| Pyrophosphatase | 2 µL |
| RNase inhibitor | 2 µL |
| T7 RNA Polymerase | 2 µL |
| RNANuclease free Water | Total 20 µL |

Incubation was carried out at 37°C for 2 to 4 hours, 2 µL of DNaseI was added for digestion at 37°C for 15 minutes.

### 2) Purification of transcript mRNA

The foregoing obtained transcript was purified via a silica spin column method (Thermo, GeneJET RNA Purification Kit), and the OD value was measured and 1% denatured agarose gel electrophoresis was used to identify an RNA size (FIG. 1 to FIG. 3). Figures of denatured agarose gel electrophoresis shown in FIG. 1 to FIG. 3 revealed that the linear mRNA and the circular RNA were successfully synthesized, and the mRNA in the cyclization treatment group migrated faster on the gel than the linear mRNA, and the band was cyclized completely.

### 2.2.4 Transfection of 293T cells with circular mRNA encoding EGFP and measurement of fluorescence intensity

2.2.4.1 Cell culture: 293T cells were inoculated in a DMEM high-glucose medium containing 10% fetal bovine serum and 1% double antibody, and incubated at 37°C in a 5% CO₂ incubator. Subculture of cells was carried out every other 2-3 days.

2.2.4.2 Cell transfection: before transfection, the 293T cells were seeded in a 24-well plate at 1×10⁵ cells/well, and incubated at 37°C in a 5% CO₂ incubator. After a confluence of the cells reached 70% to 90%, a transfection reagent Lipofectamine Messenger Max (Invitrogen) was used to transfect the 293T cells at 500 ng of mRNA per well. Detailed operations were as follows:

### 1) Dilution of Messenger MAX^{™} Reagent

**Table 8**

| Reagent | Volume/well |
|---|---|
| MEM serum-free medium | 25 µL |
| Messenger MAX^{™} Reagent | 0.75 µL |

Incubation was carried out by standing at room temperature for 10 minutes after dilution and mixing.

### 2) Dilution of mRNA

**Table 9**

| Reagent | Volume/well |
|---|---|
| mRNA | 500 ng |
| MEM serum-free medium | made up to 25 µL |

### 3) Selection of Mixed and Diluted Messenger MAX^{™} Reagent and mRNA (1: 1)

**Table 10**

| Reagent | Volume/well |
|---|---|
| Diluted Messenger MAX^{™} Reagent | 25 µL |
| Diluted mRNA | 25 µL |

Incubation was carried out by standing at room temperature for 5 minutes after dilution and mixing.

4) 50 µL of the above mixed solution was sucked and slowly added to the 24-well plate in an adherent manner, and incubation was carried out at 37°C in the 5% COz incubator.

### 2.2.4.3 Test of protein expression

1) Cell fluorescence observation: expression of EGFP was observed in the 293T cells 24 hours after transfection under a fluorescence microscope.
2) Test of average fluorescence intensity of cells via flow cytometry: the average fluorescence intensity of the 293T cells were measured by using a flow cytometer 24 hours after transfection.

### 2.2.5 Analysis of test results

No active IRES sequence was added to the circular mRNA molecule in the control 1, and a coxsackievirus B3 (CVB3) sequence (SEQ ID NO: 562) with high IRES activity was added to the circular mRNA molecule in the control 2. The test results are shown in the table below. If the expression level of EGFP was greater than 0 and less than or equal to 10000, it indicated that the to-be-predicted sequence mediated the expression of the circular RNA, and contained the IRES sequence; if the expression level of EGFP is greater than 10000, it indicated that the IRES contained in the to-be-predicted sequence had extremely good activity.

**Table 11**

| SEQ ID NO: | eGFP expres sion level | SEQ ID NO: | eGFP expressio n level | SEQ ID NO: | eGFP expressio n level | SEQ ID NO: | eGFP expres sion level |
|---|---|---|---|---|---|---|---|
| Control 1 | 0 | 139 | 15590 | 278 | 21532 | 417 | 1272 |
| 1 | 29221 | 140 | 601 | 279 | 20713 | 418 | 1043 |
| 2 | 17075 | 141 | 6431 | 280 | 23898 | 419 | 736 |
| 3 | 29269 | 142 | 12100 | 281 | 21122 | 420 | 506 |
| 4 | 20991 | 143 | 5926 | 282 | 20382 | 421 | 1019 |
| 5 | 12371 | 144 | 9023 | 283 | 18398 | 422 | 6791 |
| 6 | 9263 | 145 | 6053 | 284 | 22921 | 423 | 1505 |
| 7 | 10301 | 146 | 5527 | 285 | 22987 | 424 | 1111 |
| 8 | 11887 | 147 | 6638 | 286 | 17122 | 425 | 511 |
| 9 | 14138 | 148 | 9410 | 287 | 17989 | 426 | 381 |
| 10 | 25237 | 149 | 4890 | 288 | 11270 | 427 | 436 |
| 11 | 35087 | 150 | 5021 | 289 | 16458 | 428 | 345 |
| 12 | 7557 | 151 | 2678 | 290 | 8700 | 429 | 931 |
| 13 | 29810 | 152 | 8172 | 291 | 23033 | 430 | 591 |
| 14 | 26472 | 153 | 6613 | 292 | 12443 | 431 | 7789 |
| 15 | 22694 | 154 | 4961 | 293 | 21616 | 432 | 6651 |
| 16 | 12621 | 155 | 5161 | 294 | 22761 | 433 | 703 |
| 17 | 31332 | 156 | 8514 | 295 | 7891 | 434 | 5589 |
| 18 | 22290 | 157 | 349 | 296 | 45345 | 435 | 478 |
| 19 | 23429 | 158 | 8106 | 297 | 3891 | 436 | 17046 |
| 20 | 25904 | 159 | 11662 | 298 | 34488 | 437 | 349 |
| 21 | 887 | 160 | 4213 | 299 | 9871 | 438 | 13995 |
| 22 | 12438 | 161 | 7910 | 300 | 511 | 439 | 17677 |
| 23 | 728 | 162 | 11675 | 301 | 36127 | 440 | 11416 |
| 24 | 3451 | 163 | 280 | 302 | 27811 | 441 | 18705 |
| 25 | 23699 | 164 | 7944 | 303 | 24601 | 442 | 7761 |
| 26 | 25696 | 165 | 19436 | 304 | 25929 | 443 | 355 |
| 27 | 32602 | 166 | 11313 | 305 | 34899 | 444 | 9489 |
| 28 | 23039 | 167 | 11189 | 306 | 31458 | 445 | 24062 |
| 29 | 399 | 168 | 12517 | 307 | 32755 | 446 | 5561 |
| 30 | 343 | 169 | 11698 | 308 | 33312 | 447 | 4798 |
| 31 | 354 | 170 | 9133 | 309 | 18319 | 448 | 2289 |
| 32 | 8365 | 171 | 7366 | 310 | 13233 | 449 | 622 |
| 33 | 11190 | 172 | 11427 | 311 | 14579 | 450 | 9617 |
| 34 | 10725 | 173 | 11991 | 312 | 24613 | 451 | 2391 |
| 35 | 10890 | 174 | 1789 | 313 | 4040 | 452 | 5581 |
| 36 | 11818 | 175 | 2368 | 314 | 25067 | 453 | 7819 |
| 37 | 10761 | 176 | 5525 | 315 | 22954 | 454 | 8910 |
| 38 | 7885 | 177 | 3356 | 316 | 7653 | 455 | 6719 |
| 39 | 10150 | 178 | 4578 | 317 | 21439 | 456 | 1375 |
| 40 | 322 | 179 | 17780 | 318 | 21495 | 457 | 14380 |
| 41 | 13604 | 180 | 15827 | 319 | 20583 | 458 | 8024 |
| 42 | 13239 | 181 | 7890 | 320 | 9556 | 459 | 7045 |
| 43 | 12396 | 182 | 12115 | 321 | 17712 | 460 | 13124 |
| 44 | 11558 | 183 | 15495 | 322 | 14206 | 461 | 706 |
| 45 | 20827 | 184 | 11875 | 323 | 20070 | 462 | 2144 |
| 46 | 29790 | 185 | 1235 | 324 | 25019 | 463 | 4141 |
| 47 | 12569 | 186 | 13625 | 325 | 3312 | 464 | 868 |
| 48 | 11001 | 187 | 4356 | 326 | 17706 | 465 | 553 |
| 49 | 7534 | 188 | 13462 | 327 | 12655 | 466 | 9810 |
| 50 | 9704 | 189 | 10415 | 328 | 726 | 467 | 325 |
| 51 | 13760 | 190 | 6798 | 329 | 13420 | 468 | 354 |
| 52 | 11911 | 191 | 7508 | 330 | 884 | 469 | 308 |
| 53 | 12251 | 192 | 9261 | 331 | 25557 | 470 | 651 |
| 54 | 9974 | 193 | 8485 | 332 | 16937 | 471 | 9810 |
| 55 | 10235 | 194 | 6625 | 333 | 16868 | 472 | 5561 |
| 56 | 14185 | 195 | 6051 | 334 | 21053 | 473 | 8771 |
| 57 | 12646 | 196 | 8719 | 335 | 15213 | 474 | 2718 |
| 58 | 3452 | 197 | 6394 | 336 | 27120 | 475 | 1981 |
| 59 | 21316 | 198 | 20029 | 337 | 6088 | 476 | 2718 |
| 60 | 3421 | 199 | 10627 | 338 | 4579 | 477 | 845 |
| 61 | 400 | 200 | 22761 | 339 | 5801 | 478 | 2371 |
| 62 | 10943 | 201 | 10673 | 340 | 11110 | 479 | 2718 |
| 63 | 10299 | 202 | 5240 | 341 | 2317 | 480 | 819 |
| 64 | 10455 | 203 | 4538 | 342 | 8965 | 481 | 3231 |
| 65 | 7979 | 204 | 6008 | 343 | 6543 | 482 | 2718 |
| 66 | 11583 | 205 | 7355 | 344 | 9947 | 483 | 327 |
| 67 | 9016 | 206 | 5444 | 345 | 6014 | 484 | 399 |
| 68 | 281 | 207 | 5808 | 346 | 7891 | 485 | 579 |
| 69 | 6117 | 208 | 8509 | 347 | 4497 | 486 | 2585 |
| 70 | 1456 | 209 | 4643 | 348 | 14524 | 487 | 7819 |
| 71 | 9746 | 210 | 7374 | 349 | 5541 | 488 | 4830 |
| 72 | 13013 | 211 | 4270 | 350 | 5020 | 489 | 5247 |
| 73 | 278 | 212 | 4949 | 351 | 5561 | 490 | 2695 |
| 74 | 7892 | 213 | 4379 | 352 | 5504 | 491 | 1221 |
| 75 | 5470 | 214 | 7689 | 353 | 6781 | 492 | 2819 |
| 76 | 7721 | 215 | 21144 | 354 | 11487 | 493 | 292 |
| 77 | 841 | 216 | 27823 | 355 | 6747 | 494 | 10472 |
| 78 | 8171 | 217 | 24799 | 356 | 7981 | 495 | 343 |
| 79 | 19209 | 218 | 21715 | 357 | 4292 | 496 | 20591 |
| 80 | 310 | 219 | 20302 | 358 | 2451 | 497 | 1819 |
| 81 | 4328 | 220 | 22281 | 359 | 1677 | 498 | 8838 |
| 82 | 5306 | 221 | 18407 | 360 | 4517 | 499 | 11717 |
| 83 | 5055 | 222 | 25004 | 361 | 5023 | 500 | 8460 |
| 84 | 8931 | 223 | 30001 | 362 | 9642 | 501 | 8910 |
| 85 | 7222 | 224 | 3219 | 363 | 7575 | 502 | 2359 |
| 86 | 5289 | 225 | 26036 | 364 | 6718 | 503 | 11024 |
| 87 | 6324 | 226 | 5430 | 365 | 11587 | 504 | 13799 |
| 88 | 5609 | 227 | 26036 | 366 | 9871 | 505 | 12515 |
| 89 | 6388 | 228 | 26016 | 367 | 5670 | 506 | 11636 |
| 90 | 1975 | 229 | 26089 | 368 | 5435 | 507 | 14272 |
| 91 | 23641 | 230 | 25480 | 369 | 9277 | 508 | 2670 |
| 92 | 6765 | 231 | 26082 | 370 | 8262 | 509 | 13921 |
| 93 | 8276 | 232 | 28353 | 371 | 7612 | 510 | 719 |
| 94 | 9418 | 233 | 20880 | 372 | 6362 | 511 | 12724 |
| 95 | 9018 | 234 | 27128 | 373 | 9639 | 512 | 879 |
| 96 | 481 | 235 | 22492 | 374 | 1582 | 513 | 6719 |
| 97 | 7920 | 236 | 16527 | 375 | 3365 | 514 | 15459 |
| 98 | 24446 | 237 | 3345 | 376 | 8912 | 515 | 2376 |
| 99 | 8317 | 238 | 1242 | 377 | 7983 | 516 | 12313 |
| 100 | 1256 | 239 | 27797 | 378 | 3850 | 517 | 2367 |
| 101 | 24473 | 240 | 14851 | 379 | 9871 | 518 | 3121 |
| 102 | 4762 | 241 | 4378 | 380 | 6694 | 519 | 287 |
| 103 | 5051 | 242 | 17024 | 381 | 7829 | 520 | 4214 |
| 104 | 25717 | 243 | 24485 | 382 | 10159 | 521 | 836 |
| 105 | 6133 | 244 | 25463 | 383 | 10299 | 522 | 4567 |
| 106 | 15307 | 245 | 17626 | 384 | 7369 | 523 | 6741 |
| 107 | 14202 | 246 | 25950 | 385 | 21244 | 524 | 4321 |
| 108 | 2235 | 247 | 17476 | 386 | 2641 | 525 | 4521 |
| 109 | 370 | 248 | 41579 | 387 | 13758 | 526 | 2513 |
| 110 | 24772 | 249 | 47535 | 388 | 10082 | 527 | 3421 |
| 111 | 281 | 250 | 30143 | 389 | 13306 | 528 | 10198 |
| 112 | 6786 | 251 | 33693 | 390 | 8735 | 529 | 303 |
| 113 | 2127 | 252 | 36779 | 391 | 12278 | 530 | 406 |
| 114 | 593 | 253 | 43377 | 392 | 14340 | 531 | 6521 |
| 115 | 17246 | 254 | 41163 | 393 | 15015 | 532 | 343 |
| 116 | 20619 | 255 | 26784 | 394 | 18180 | 533 | 320 |
| 117 | 18487 | 256 | 20119 | 395 | 12864 | 534 | 24948 |
| 118 | 14381 | 257 | 36914 | 396 | 9541 | 535 | 2231 |
| 119 | 19184 | 258 | 39011 | 397 | 6549 | 536 | 3952 |
| 120 | 7689 | 259 | 5627 | 398 | 10594 | 537 | 446 |
| 121 | 3438 | 260 | 8917 | 399 | 12189 | 538 | 338 |
| 122 | 14187 | 261 | 24495 | 400 | 9871 | 539 | 307 |
| 123 | 19131 | 262 | 39506 | 401 | 8324 | 540 | 3410 |
| 124 | 2367 | 263 | 38283 | 402 | 9651 | 541 | 371 |
| 125 | 21467 | 264 | 38788 | 403 | 10626 | 542 | 314 |
| 126 | 285 | 265 | 41324 | 404 | 9490 | 543 | 306 |
| 127 | 27497 | 266 | 34856 | 405 | 9014 | 544 | 274 |
| 128 | 4110 | 267 | 39125 | 406 | 14962 | 545 | 3421 |
| 129 | 20264 | 268 | 42832 | 407 | 898 | 546 | 363 |
| 130 | 16132 | 269 | 36835 | 408 | 845 | 547 | 351 |
| 131 | 5910 | 270 | 35262 | 409 | 8910 | 548 | 307 |
| 132 | 9565 | 271 | 4517 | 410 | 771 | Control 2 | 12692 |
| 133 | 3980 | 272 | 25974 | 411 | 1071 | | |
| 134 | 394 | 273 | 17804 | 412 | 561 | | |
| 135 | 21244 | 274 | 19160 | 413 | 355 | | |
| 136 | 2891 | 275 | 22032 | 414 | 840 | | |
| 137 | 315 | 276 | 21567 | 415 | 720 | | |
| 138 | 9187 | 277 | 8337 | 416 | 329 | | |

It could be learned from the above Table 11 that the polynucleotides of the sequences shown in the SEQ ID NOs: 1 to 548 in the disclosure all had the activity of initiating protein translation of the circular mRNA molecule, and could be used as the IRES element to construct a circular mRNA molecule having protein and polypeptide translation activity. In some preferred embodiments, the EGFP expression level of the circular mRNA molecules constructed by using the polynucleotide in the disclosure was higher than that of the circular nucleic acid molecule constructed by using Coxsackievirus B3 (CVB3) (shown in SEQ ID NO: 562), indicating that the IRES activity of the polynucleotide provided by the disclosure was further improved compared with the current highly-active IRES sequence, which was of great significance for improving the levels of expressing the protein of interest and the polypeptide of interest by the circular nucleic acid molecule.

All technical features disclosed in this specification can be combined in any manner. Each feature disclosed in this specification may also be replaced with other features having the same, equivalent or similar function. Therefore, unless otherwise specified, each disclosed feature is only an instance of a series of equivalent or similar features.

In addition, from the foregoing descriptions, a person skilled in the art can easily learn a key feature of the present invention, and can make many modifications to the invention to adapt to various use purposes and conditions without departing from the spirit and scope of the present invention. Therefore, such modifications are also intended to fall within the scope of the appended claims.

## Claims

1. A Levenshtein distance-based internal ribosome entry site (IRES) screening method, comprising the following steps:
(1) selecting n sequences comprising an IRES as sample sequences, wherein n≥1 and n is a natural number;
(2) subjecting the sample sequences and to-be-predicted sequences to one-hot encoding respectively, wherein categorical variables are A, T, C, and G;
(3) traversing the sample sequences, and calculating a Levenshtein distance between each sample sequence and the to-be-predicted sequence;
(4) calculating an average of Levenshtein distances between all sample sequences and the to-be-predicted sequences; and
(5) determining, based on the average, whether the to-be-predicted sequences comprise the IRES.

2. The Levenshtein distance-based IRES screening method according to claim 1, wherein in the step (5), if the average is not less than a set prediction threshold, it is determined that the to-be-predicted sequence comprises the IRES, otherwise it is determined that the to-be-predicted sequence comprises no IRES.

3. The Levenshtein distance-based IRES screening method according to claim 2, wherein the prediction threshold is not less than 0.5, and optionally, the prediction threshold is 0.75.

4. The Levenshtein distance-based IRES screening method according to any one of claims 1 to 3, wherein the method further comprises the following step: subjecting a to-be-predicted sequence determined to comprise the IRES to experimental verification to verify the IRES activity of the to-be-predicted sequence.

5. The Levenshtein distance-based IRES screening method according to claim 4, wherein the experimental verification comprises the steps of:
constructing a circular nucleic acid molecule by using the to-be-predicted sequence determined to comprise the IRES, wherein in the circular nucleic acid molecule, the to-be-predicted sequence is operably linked to a nucleotide sequence encoding a fluorescent protein; and
obtaining a fluorescence signal released by the circular nucleic acid molecule, and determining the IRES activity of the to-be-predicted sequence based on the fluorescence signal.

6. A polynucleotide, wherein the polynucleotide is selected from at least one of the group consisting of (i) to (iv):
(i) comprising a nucleotide sequence shown in any one of SEQ ID NOs: 1, 2, 3, 4, 9, 10, 11, 13, 14, 15, 17, 18, 19, 20, 25, 26, 27, 28, 41, 42, 45, 46, 51, 56, 59, 72, 79, 91, 98, 101, 104, 106, 107, 110, 115, 116, 117, 118, 119, 122, 123, 125, 127, 129, 130, 135, 139, 165, 179, 180, 183, 186, 188, 198, 200, 215, 216, 217, 218, 219, 220, 221, 222, 223, 225, 227, 228, 229, 230, 231, 232, 233, 234, 235, 236, 239, 240, 242, 243, 244, 245, 246, 247, 248, 249, 250, 251, 252, 253, 254, 255, 256, 257, 258, 261, 262, 263, 264, 265, 266, 267, 268, 269, 270, 272, 273, 274, 275, 276, 278, 279, 280, 281, 282, 283, 284, 285, 286, 287, 289, 291, 293, 294, 296, 298, 301, 302, 303, 304, 305, 306, 307, 308, 309, 310, 311, 312, 314, 315, 317, 318, 319, 321, 322, 323, 324, 326, 329, 331, 332, 333, 334, 335, 336, 348, 385, 387, 389, 392, 393, 394, 395, 406, 436, 438, 439, 441, 445, 457, 460, 496, 504, 507, 509, 511, 514, and 534;
(ii) a mutant sequence of any one nucleotide sequence shown in (i), wherein the mutant sequence has a mutant nucleotide at one or more positions of any corresponding nucleotide sequence shown in (i), and the mutant sequence has an activity of initiating translation of a circular nucleic acid molecule;
(iii) a nucleotide sequence that can be reversely complementary to a hybridized sequence of the nucleotide sequence shown in (i) or (ii) under a highly stringent hybridization condition or a very highly stringent hybridization condition and that has an activity of initiating translation of a circular nucleic acid molecule; and
(iv) a nucleotide sequence having at least 70%, optionally at least 80%, preferably at least 90%, more preferably at least 95%, most preferably at least 98% sequence identity with the nucleotide sequence shown in any one of (i) or (ii) and having an activity of initiating translation of a circular nucleic acid molecule.

7. The polynucleotide according to claim 6, wherein the polynucleotide is a polynucleotide comprising the IRES that is screened by the method according to any one of claims 1 to 5.

8. Use of the polynucleotide according to claim 6 or 7 in at least one of (a₁)-(a₂):
(a₁) initiating translation of a circular nucleic acid molecule, or preparing a product for initiating translation of a circular nucleic acid molecule; and
(a₂) increasing a protein expression level of a circular nucleic acid molecule, or preparing a product for increasing a protein expression level of a circular nucleic acid molecule.

9. A circular nucleic acid molecule, wherein the circular nucleic acid molecule comprises the polynucleotide according to claim 6 or 7;
preferably, the circular nucleic acid molecule further comprises a coding region encoding a polypeptide of interest, and the coding region is operably linked to the polynucleotide; and
optionally, the circular nucleic acid molecule further comprises one or more of the following elements: a 5' spacer region, a 3' spacer region, a second exon, and a first exon.

10. A cyclization precursor nucleic acid molecule, wherein the cyclization precursor nucleic acid molecule is cyclized to form the circular nucleic acid molecule according to claim 9; and
optionally, the cyclization precursor nucleic acid molecule further comprises one or more of the following elements:
a 5' homology arm, a 3' intron, a second exon, a 5' spacer region, a coding region, a 3' spacer region, a first exon, a 5' intron and a 3' homology arm.

11. A recombinant nucleic acid molecule, wherein the recombinant nucleic acid molecule is selected from any one of (f₁)-(f₂):
(f₁) comprising the polynucleotide according to claim 6 or 7; and
(f₂) transcription to form the cyclization precursor nucleic acid molecule according to claim 10.

12. A recombinant expression vector, wherein the recombinant expression vector comprises the recombinant nucleic acid molecule according to claim 11.

13. A recombinant host cell, wherein the recombinant host cell comprises the polynucleotide according to claim 6 or 7, the circular nucleic acid molecule according to claim 9, the cyclization precursor nucleic acid molecule according to claim 10, the recombinant nucleic acid molecule according to claim 11, or the recombinant expression vector according to claim 12.

14. A method for preparing a circular nucleic acid molecule with an improved protein expression level, wherein the method comprises a step of operably linking the polynucleotide according to claim 6 or 7 to a coding region of the circular nucleic acid molecule.

15. Use of the circular nucleic acid molecule according to claim 9, the cyclization precursor nucleic acid molecule according to claim 10, the recombinant nucleic acid molecule according to claim 11, or the recombinant expression vector according to claim 12 in at least one of (g₁) to (g₃):
(g₁) expressing a protein, or preparing a product for expressing a protein;
(gz) expressing a polypeptide, or preparing a product for expressing a polypeptide; and
(g₃) serving as or preparing a nucleic acid vaccine;
optionally, the protein or the polypeptide is one or more selected from: an antigen, an antibody, an antigen-binding fragment, a channel protein, a receptor, a cytokine, and an immune checkpoint inhibitor.
